# DEMANDE DE BREVET EUROPEEN

(11) **EP 4 389 136 A1**
(43) Date de publication de la demande: **26.06.2024**
(21) Numéro de dépôt: 23218207.1
(22) Date de dépôt: 19.12.2023
(51) Int. Cl.: A61K 36/48, A61K 36/539, A61K 36/889, A61K 8/9789, A61Q 19/00, A61Q 19/08, A61P 17/18, A61P 17/00, A61P 17/06, A61Q 19/02, A61Q 17/00

(54) **COMPOSITION NEUROCOSMETIQUE POUR PREVENIR LES EFFETS DU STRESS**

(30) Priorité: 20.12.2022 FR 2214015
(71) Demandeur: C.F.E.B. Sisley, 75008 Paris (FR)
(72) Inventeur: GINESTAR, José, 95800 COURDIMANCHE (FR); THUILLIER, Isabelle, 75008 PARIS (FR); BERTRAND, Caroline, 95290 L'ISLE ADAM (FR); ANDRE, Nada, 78580 MAULE (FR)
(74) Mandataire: Regimbeau

(57) **Abrégé**

La présente invention a pour objet l'utilisation d'une composition neurocosmétique cutanée permettant à ses utilisateurs de prévenir les effets du stress sur leur peau, et de procurer une sensation de bien-être. Les présents inventeurs ont en effet identifié qu'une combinaison particulière de deux plantes, à savoir la noix de coco et la scutellaire des Alpes, limite les effets cutanés délétères induits par les situations de stress chronique ou ponctuel. Plus précisément, la composition de l'invention, contenant une quantité efficace de noix de coco et de scutellaire des Alpes (*Scutellaria alpina*), et optionnellement un extrait d'écorce *d'Eperua falcata,* inhibe la libération de neuropeptides due à l'activation des neurones sensitifs cutanés en situation de stress, augmente l'expression des récepteurs aux opioïdes µ (MOR) et limite l'augmentation due au stress des récepteurs aux glucocorticoïdes (GR) à la surface de ces neurones, augmente leur survie et la taille de leurs neurites, à l'état basal ou en situation de stress. De manière avantageuse, l'effet neurosensoriel bénéfique observé pour la composition de l'invention est capable de surpasser l'effet de la molécule beta-endorphine utilisée comme contrôle.

## Description

### RESUME DE L'INVENTION

La présente invention a pour objet l'utilisation d'une composition neurocosmétique cutanée permettant à ses utilisateurs de prévenir les effets du stress sur leur peau, et de procurer une sensation de bien-être. Les présents inventeurs ont en effet identifié qu'une combinaison particulière de deux plantes, à savoir la noix de coco et la scutellaire des Alpes, limite les effets cutanés délétères induits par les situations de stress chronique ou ponctuel. Plus précisément, la composition de l'invention, contenant une quantité efficace de noix de coco et de scutellaire des Alpes (*Scutellaria alpina*), et optionnellement un extrait d'écorce *d'Eperua falcata,* inhibe la libération de neuropeptides due à l'activation des neurones sensitifs cutanés en situation de stress, augmente l'expression des récepteurs aux opioïdes µ (MOR) et limite l'augmentation due au stress des récepteurs aux glucocorticoïdes (GR) à la surface de ces neurones, augmente leur survie et la taille de leurs neurites, à l'état basal ou en situation de stress. De manière avantageuse, l'effet neurosensoriel bénéfique observé pour la composition de l'invention est capable de surpasser l'effet de la molécule beta-endorphine utilisée comme contrôle.

### DESCRIPTION DE L'ART ANTERIEUR

La peau constitue une barrière contre les agressions extérieures, notamment chimiques, mécaniques ou infectieuses, et à ce titre un certain nombre de réactions de défense contre les facteurs environnementaux (climat, rayons ultra violets, tabac) et/ou les agents toxiques, comme par exemple les micro-organismes ou les xénobiotiques, se produisent à son niveau.

La peau est constituée principalement de deux compartiments, l'un superficiel, l'épiderme, et un plus profond, le derme, qui interagissent. L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau, notamment le rôle de protection de l'organisme des agressions extérieures appelé "fonction barrière".

Le Professeur L. Misery a été l'un des premiers à s'intéresser à la relation entre la peau et le cerveau en introduisant le terme de système « neuro-immuno-cutané » en 1996. Il définissait le terme « neurocosmétique » comme un produit cosmétique ayant la possibilité d'intervenir sur les cellules cutanées en modulant ce système neuro-immuno-cutané.

Depuis, il a été décrit dans la littérature que différents types cellulaires présents au niveau cutané, et en particulier les kératinocytes humains qui constituent l'un des composants essentiels de l'épiderme, sont capables de synthétiser et de sécréter de la bêta-endorphine (β-endorphine). La β-endorphine, produite localement, est capable d'exercer des effets biologiques bénéfiques, locaux ou généraux, sur les neurones sensoriels présents dans la peau (dans l'épiderme et dans le derme), qui expriment les récepteurs aux opioïdes. Par ailleurs, les neurones cutanés sont également capables de sécréter de la β-endorphine pour modifier la migration et la différenciation des kératinocytes, ainsi que la sécrétion de cytokines par ces cellules (Bigliardi-Qi et al, Dermatology. 2004,209(3): 183-9)*.* A ce sujet, la demande FR 2857588 (L'OREAL) décrit l'utilisation de la β-endorphine et de fragments actifs de cette protéine ou d'un agent capable de mimer l'activité de cette protéine, pour favoriser la différenciation kératinocytaire et la cornéification des kératinocytes, afin d'améliorer l'hydratation de la peau, lutter contre l'altération de la barrière cutanée à la suite de stress externes (effets des polluants atmosphériques, des rayonnements ultra-violets) ou de stress internes (psychologiques). Les inventeurs de FR 2857588 ont notamment démontré qu'un extrait de fève de cacao riche en polyphénol et en bases xanthiques, en particulier en théobromine, a un profil d'activité comparable à celui de la β-endorphine et procure un effet hydratant significatif après 4 semaines d'application sur la peau (exemple 4).

Les neurones sensoriels, tout comme les cellules de l'épiderme, sécrètent de multiples substances qui nourrissent un dialogue permanent entre la peau et le système nerveux. Par ces liens étroits, le système nerveux participe activement et significativement au maintien de l'équilibre cutané.

Dans ce contexte, l'objectif des présents inventeurs était d'identifier des actifs naturels (i.e., des extraits de plantes) capables de stimuler les neurones sensitifs cutanés de manière encore plus forte que la β-endorphine, afin de proposer une composition neurocosmétique présentant une meilleure activité relaxante et apaisante que celles décrites dans l'art antérieur.

### DESCRIPTION DETAILLEE DE L'INVENTION

Dans cette logique, les présents inventeurs ont identifié deux plantes dont les propriétés de stimulation des neurones sensitifs cutanés n'avaient jamais été décrites antérieurement. La combinaison de ces deux plantes est susceptible de produire un effet apaisant sur la peau, de participer au bien-être sensoriel de l'utilisateur, et de lutter contre le stress.

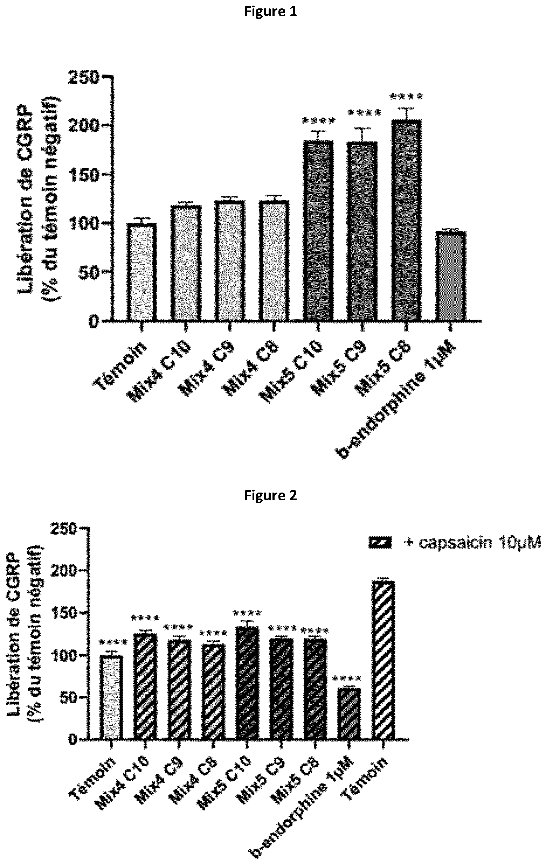

Ces deux plantes sont la noix de coco et la scutellaire des Alpes (*Scutellaria alpina*), dont il est ici démontré que la combinaison limite les effets cutanés délétères induits par les situations de stress chronique ou ponctuel, au niveau des kératinocytes et des neurones. Plus précisément, la combinaison de plantes de l'invention inhibe la libération du neuropeptide appelé « Calcitonin Gene-Related Peptide » (CGRP) lors de l'activation des neurones sensitifs cutanés en situation de stress, augmente l'expression des récepteurs aux opioïdes µ (MOR) et inhibe l'expression post-stress des récepteurs aux glucocorticoïdes (GR) à la surface de ces neurones, augmente leur survie et la taille de leurs neurites, que ce soit à l'état basal ou en situation de stress (cf. figures 1-20).

De manière surprenante, l'effet neurosensoriel mesuré dans les expériences présentées ci-dessous est capable de surpasser l'effet bénéfique de la molécule bêta-endorphine (cf. figures 1-20, la bêta-endorphine ayant été utilisée comme contrôle).

Les inventeurs démontrent en outre dans les exemples ci-dessous qu'il est possible d'augmenter plus encore l'effet neurosensoriel de cette combinaison de plantes, en ajoutant à celle-ci un extrait *d'Eperua falcata.* Ils proposent donc, dans un mode de réalisation particulier, une composition à activité neurocosmétique contenant trois extraits végétaux : de la noix de coco, de la scutellaire des Alpes ainsi que de *l'Eperua falcata.*

### Composition de l'invention

Les présents inventeurs démontrent, dans la partie expérimentale ci-dessous, l'effet d'une composition selon l'invention qui contient un extrait de fruit de *cocos nucifera* (extrait de noix de coco) et un extrait de *Scutellaria alpina* (scutellaire des alpes) (composition appelée « Mix 4 » dans les exemples).

Leurs résultats suggèrent que cette composition possède des propriétés apaisantes permettant de contrer l'effet irritant de la capsaïcine et du cortisol. En effet, au niveau cellulaire, elle améliore la physiologie des neurones cutanés en augmentant leur survie, en activant leur pousse neuritique et en augmentant l'expression des µ-opioïdes récepteur (MOR) et en limitant l'expression post-stress des récepteurs aux glucocorticoïdes (GR) par ces cellules. Plus précisément, comme en témoignent les résultats présentés sur les figures 1-20, aux trois concentrations testées (C8, C9 et C10), le « Mix 4 » permet i) d'améliorer la survie et d'augmenter la pousse neuritique des neurones sensitifs de la peau en présence d'un stress, ii) d'inhiber la toxicité due au traitement des neurones par le cortisol ou la capsaïcine, iii) d'augmenter l'expression des µ-opioïdes récepteurs (récepteurs MOR) à la surface des neurones et d'inhiber l'effet délétère du cortisol sur le taux d'expression de ces récepteurs, iv) de limiter l'augmentation de l'expression post-stress des récepteurs aux glucocorticoïdes (GR) par les neurones sensitifs et d'inhiber l'effet délétère du cortisol sur le taux d'expression de ces GR et v) d'inhiber significativement la libération de neuropeptides CGRP induite par le stress (capsaïcine ou cortisol). Ces effets dépassent fréquemment l'effet de la bêta-endorphine, utilisée ici comme contrôle.

Ainsi, dans un premier aspect, la présente invention concerne une composition neurocosmétique à usage topique, comprenant :
a) Une quantité efficace d'un extrait de fruit de *Cocos nucifera,* et
b) Une quantité efficace d'un extrait de *Scutellaria alpina.*

Cette composition est une composition « neurocosmétique » au sens proposé par M. Misery en 2000, à savoir un produit qui, lorsqu'il est appliqué sur la peau, présente une activité sur le système nerveux cutané ou des effets généraux sur les médiateurs de la peau.

Les inventeurs ont également constaté que l'effet neurostimulant était augmenté lorsqu'un extrait d'écorce *d'Eperua falcata* était ajouté au « Mix 4 » décrit ci-dessus. En effet, comme en témoignent les résultats présentés sur les figures 1-20, l'effet du « Mix 5 », qui contient des extraits de *Cocos nucifera,* de *Scutellaria alpina et* d'*Eperua falcata,* est supérieur à celui du « Mix 4 » en ce qui concerne la pousse neuritique (figure 6), y compris après l'application d'un stress (figures 10, 14 et 18).

Dans un mode de réalisation préférentiel, la composition de l'invention contient donc, en plus des extraits de noix de coco et de Scutellaire des Alpes, une quantité efficace d'un extrait *d'Eperua falcata.*

Les compositions de l'invention contiennent donc au moins de la noix de coco et de la Scutellaire des Alpes (et optionnellement un extrait d'écorce *d'Eperua falcata*)*.* Elles possèdent des propriétés apaisantes permettant de contrer l'effet irritant d'un stress cutané aigu ou chronique. Elles améliorent la physiologie des neurones en augmentant leur survie, en activant la pousse neuritique, en augmentant l'expression des récepteurs aux µ-opioïdes (MOR) et en limitant l'expression post-stress des récepteurs aux glucocorticoïdes (GR). Globalement, elles sont donc capables de protéger les neurones de l'effet délétère d'un stress cutané, aigu ou chronique.

Les différents ingrédients présents dans la composition de l'invention sont décrits plus en détail ci-dessous.

### Cocos nucifera

Le cocotier (*Cocos nucifera*) est une espèce de palmiers de la famille des *Arecaceae.* Son fruit est la noix de coco : ovale et dur, vert ou jaune, il mesure entre 10 et 40 cm de long et entre 10 et 16 cm de large. Il pèse jusqu'à 1,5 kg et apparaît sur une spathe entre les longues feuilles pennées ; sa graine a une enveloppe brune, fibreuse ; sa chair blanche (albumen) fraîche ou séchée (coprah) est comestible, ainsi que l'eau de coco.

La composition de l'invention contient une quantité efficace d'un « extrait de noix de coco ».

L'intérieur de la noix de coco est rempli d'un liquide appelé « eau de coco » tandis que la pulpe contient de « l'huile de coco ». Les extraits de noix de coco, d'huile de coco, d'eau de coco et de crème de noix de coco sont couramment utilisés dans les produits cosmétiques pour leurs effets hydratants, régénérants et apaisants. L'eau de coco est riche en antioxydants qui protègent la peau contre les effets extérieurs défavorables. Elle contient également des vitamines, des minéraux, des cytokinines qui stimulent la croissance des cellules et de l'acide laurique qui aide à freiner le vieillissement de la peau et à réduire l'acné. De par sa composition, l'huile de coco est semblable aux graisses cutanées, ce qui lui permet de facilement s'absorber. Elle contient une grande quantité d'antioxydants qui luttent contre le vieillissement de la peau et les rides. Elle convient parfaitement aux peaux sèches, sensibles et matures, et elle est fortement conseillée pour le soin des peaux eczémateuses et du psoriasis. Ses effets adoucissants sont bénéfiques à la peau du corps entier, mais aussi aux cheveux. La farine de noix de coco est également obtenue à partir de la pulpe de noix de coco. Elle sert à aromatiser les gâteaux et autres desserts, mais également dans des compositions cosmétiques, en tant qu'exfoliant cutané.

Dans la composition de l'invention, les inventeurs proposent d'utiliser non pas de l'eau de coco, ni de l'huile de coco, mais de la farine de noix de coco. Ainsi, par « extrait de noix de coco », on entend ici de préférence de la farine de noix de coco. Cette farine peut être obtenue par n'importe quel moyen classique (séchage, dégraissage par pression, puis broyage de la chair de noix de coco).

De manière encore plus préférée, dans la composition de l'invention, les inventeurs proposent d'utiliser l'ingrédient Sensorialine^{™} vendu par Silab en tant « extrait de noix de coco ». Cet ingrédient, obtenu à partirde farine de noix de coco, est connu pour renforcer la fonction barrière de la peau, et pour en améliorer l'hydratation. Il est issu de la pression à froid de la chair blanche de noix de coco. La sensorialine^{™} contient des glycolipides, du butylène glycol d'origine végétale (10%), des stabilisants tels que le 1,2 hexanediol et le caprylyl glycol, de l'eau, et de la farine de noix de coco (2.6%).

L'ingrédient SENSORIALINE^{®} est composé majoritairement de sucres (73% de l'actif) et de cendres (22% de l'actif). Plus précisément, la fraction active est riche en disaccharides et disaccharides lipidiques, de glycolipides de type nonioside D et butyl-4-O-alpha-D-glucopyranosyl-beta-D-glucopyranoside.

La composition neurocosmétique de l'invention contient avantageusement entre 0.01 et 1% en poids de farine de fruit de *Cocos nucifera,* de préférence entre 0.01 et 0.5% en poids de farine de fruit de *Cocos nucifera,* de manière encore plus préférée entre 0.02 et 0.1% en poids de farine de fruit de *Cocos nucifera.*

La composition neurocosmétique de l'invention contient par exemple entre 1 et 10% en poids de Sensorialine^{™}, de préférence entre 1 et 5% en poids de Sensorialine^{™}, de manière encore plus préférée entre 1 et 3% en poids de Sensorialine^{™}.

### Scutellaria alpina

La composition de l'invention contient en outre une quantité efficace d'un extrait de *Scutellaria alpina.*

Autrement appelée la « Scutellaire des Alpes » ou « toque des Alpes » la *Scutellaria alpina* est une plante à fleurs du genre *Scutellaria,* de la famille des *Lamiaceae,* que l'on peut trouver dans les zones rocheuses des montagnes calcaires, à des altitudes de 1400-2500m. Cette plante mesure entre 10 et 40 cm de hauteur, sa tige rameuse à base ligneuse est couchée puis ascendante. Ses feuilles ovales dentées à base arrondies sont portées par un pétiole court. Ses fleurs sont groupées en épi terminal quadrangulaire dense sur des bractées parfois violacées. Sa corolle de ses fleurs est bleu-violet, elles mesurent de 2.5 à 3 cm de long, le tube et la lèvre inférieure sont blanchâtres. Son fruit est un akène pédicellé pubescent. Contrairement aux autres scutellaires, la scutellaire des Alpes ne contient pas de baicaline et de chrisin 7-O glucuronide et n'a donc jamais été proposée pour ses vertus anxiolytiques.

La Scutellaire des Alpes contient en revanche de la scutellarine (acide 4',5,6-Trihydroxyflavon-7-yl β-D-glucopyranosiduronique) dont les vertus antioxydantes, anti-inflammatoires, sédatives, et anti-apoptotiques ont été décrites dans le passé (Sun C.Y. et al, J Cancer. 2018; 9(18): 3247-3256 ; Wang L. et al, Pharmacology & Therapeutics Volume 190, October 2018, Pages 105-127). Elle contient également des flavonoïdes, de l'apigenin-7-O-glucuronide et de la luteolin-7-O-glucuronide, dont les effets anti-oxydants et anti-inflammatoires sont connus.

De préférence, l'extrait de Scutellaire des Alpes présent dans la composition de l'invention est un extrait obtenu à partir des parties aériennes de cette plante (fleurs, feuilles, tiges et bourgeons). Cet extrait peut être obtenu par tout moyen approprié, quels que soient le procédé d'extraction, le solvant d'extraction, les composants extraits ou le type d'extrait. Cet extrait peut être notamment de la Scutellaire des Alpes non transformée (fraiche ou séchée), ou un produit issu de la pulvérisation de la plante (fraiche ou séchée), ou un produit de fermentation de la plante. La Scutellaire des Alpes utilisée dans la présente composition n'est pas nécessairement séchée et peut être sous n'importe quelle forme tant que ses ingrédients actifs peuvent être extraits de manière adéquate.

De préférence, l'extrait de Scutellaire des Alpes présent dans la composition de l'invention est obtenu en séchant puis en pulvérisant les parties aériennes de la plante, puis en utilisant un solvant d'extraction tel que l'eau ou un solvant organique choisi dans le groupe constitué d'un alcool en Ci-Ce (par exemple le méthanol ou l'éthanol), de l'acétone, de l'éther, de l'acétate d'éthyle, de l'éther diéthylique, de la méthyléthylcétone et du chloroforme, sans toutefois s'y limiter. L'extraction peut être réalisée à l'eau chaude, à l'éthanol, par chauffage, par le froid, par reflux, par condensation par reflux, par ultrasons, etc. Toute méthode d'extraction connue de l'homme du métier peut être utilisée sans limitation. Plus précisément, l'extraction peut être réalisée à l'eau chaude ou à l'éthanol. Le procédé peut en outre comprendre une étape d'élimination du solvant par distillation (par exemple sous vide) après l'extraction. Le procédé peut en outre comprendre une étape d'ajout de glycérine ou d'un autre excipient, au concentré résultant après la distillation. L'extrait peut enfin être filtré pour éliminer toute trace de résidus de plante dans le liquide résultant.

L'extraction peut être réalisée à température ambiante. Cependant, pour une extraction plus efficace, elle peut être effectuée à des températures élevées. L'extraction peut être effectuée spécifiquement à environ 40 à 100°C, plus spécifiquement à environ 80°C. L'extraction peut être effectuée pendant environ 2 à 14 heures, spécifiquement pendant environ 8 à 14 heures, plus spécifiquement pendant environ 11 à 13 heures, plus spécifiquement pendant 12 heures. Cependant, le temps d'extraction n'y est pas limité et peut varier en fonction de conditions telles que le solvant d'extraction, la température d'extraction, etc. L'extraction peut être effectuée plus d'une fois afin d'obtenir une plus grande quantité d'ingrédients actifs. L'extraction peut être effectuée en continu spécifiquement 1 à 5 fois, plus spécifiquement 3 fois, et les extraits résultants peuvent être combinés pour une utilisation ultérieure.

Dans un mode de réalisation particulier, l'extrait de Scutellaire des Alpes est obtenu selon le protocole suivant : les fleurs, feuilles et tiges de la plante sont récoltées, séchées à l'air chaud et réduites en poudre. La poudre résultante est extraite avec une solution éthanol/eau, puis l'éthanol est éliminé par distillation sous vide. Après avoir ajouté de la glycérine et un stabilisant au concentré obtenu, l'extrait est filtré.

Dans la composition de l'invention, il est possible d'utiliser par exemple l'extrait de «ALPAFLOR^{®} SCUTELLARIA CB » préparé selon la méthode décrite ci-dessus, commercialisé par DSM Nutritional Products Ltd. (4002 Bâle, Suisse). Il contient l'extrait de plante (5%), du sorbate de potassium, et de l'acide citrique. Cet ingrédient est connu pour agir sur les kératinocytes de la peau, et pour favoriser l'homéostasie de la peau. Son rôle sur les neurones cutanés n'a cependant jamais été décrit.

La composition neurocosmétique de l'invention contient avantageusement entre 0.1 et 0.5% en poids d'un extrait de fleurs, tiges et/ou feuilles de *Scutellaria alpina,* de préférence entre 0.1 et 0.3% en poids d'un extrait de fleurs, tiges et/ou feuilles de *Scutellaria alpina,* de manière encore plus préférée entre 0.1 et 0.2% en poids d'un extrait de fleurs, tiges et/ou feuilles de *Scutellaria alpina.*

La composition neurocosmétique de l'invention contient par exemple entre 1 et 10% en poids de l'Alpaflor^{®} SCUTELLARIA CB, de préférence entre 1 et 5% en poids de l'Alpaflor^{®} SCUTELLARIA CB, de manière encore plus préférée entre 2 et 4% en poids de l'Alpaflor^{®} SCUTELLARIA CB.

### Eperuline

Comme expliqué ci-dessus, la composition de l'invention contient optionnellement une quantité efficace d'un extrait *d'Eperua falcata.*

*L'Eperua falcata* est un arbre de la famille des *Caesalpiniaceae.* On le trouve notamment en Guyane française, au Vénézuela et au Brésil. Son écorce est utilisée pour les propriétés antibactérienne et antifongiques de sa résine. Ses propriétés cicatrisantes sur les blessures sont réputées. Il est également utilisé comme analgésique.

FR2786694 (SEROBIOLOGIQUES LAB SA) propose d'utiliser un extrait de cet arbre pour lutter contre le vieillissement cutané, les hyperpigmentations cutanées, les tâches de pigmentation, la perte d'élasticité de la peau, les rides, les irritations et les inflammations, la pollution, les agressions solaires et/ou dans des produits amincissants. Les parties *d'Eperua falcata* utilisées dans cette invention sont choisies parmi les racines, les écorces (des racines, des tiges et/ou du tronc des plantes), les feuilles et tiges feuillées, les fruits, les graines et/ou les fleurs de cet arbre.

La composition de la présente invention contient quant à elle de préférence (et optionnellement) une quantité efficace d'un extrait d'écorce *d'Eperua falcata.*

Après collecte, cette écorce peut être séchée puis soumise à un procédé d'extraction. Le solvant mis en oeuvre pour obtenir l'extrait de l'invention peut avantageusement être choisi dans le groupe formé par l'eau, les alcools, les cétones, les esters, les éthers, les solvants chlorés, les polyols ou les mélanges d'au moins deux solvants précités miscibles. L'extrait peut être alternativement obtenu au moyen d'un rayonnement ondulatoire, tel que les micro-ondes ou les ultrasons. L'extrait *d'Eperua falcata* peut également être obtenu par extraction avec du COz supercritique, seul ou en mélange avec un cosolvant. Selon un mode de réalisation particulier de l'invention, l'extrait *d'Eperua falcata* utilisable dans la composition de l'invention consiste en une ou des fractions isolées purifiées (notamment par chromatographie) à partir d'un extrait d'écorce *d'Eperua falcata* obtenu par un des procédés d'extraction précités.

L'extrait d'écorce *d'Eperua falcata* utile dans la composition de l'invention peut par exemple être obtenu comme suit : des écorces du tronc *d'Eperua falcata* sont concassées puis finement broyées au broyeur à couteaux. Dans un réacteur muni d'un agitateur, 3 litres d'eau distillée sont introduits et on réalise ensuite successivement les opérations suivantes consistant à : - ajouter 300 g d'écorce broyée dans le réacteur, - extraire sous agitation pendant 1 heure à ébullition, - refroidir à température ambiante, - éliminer l'insoluble par centrifugation ou filtration, - filtrer le surnageant jusqu'à une porosité d'environ 0,45 um, - recueillir le filtrat, - extraire le résidu dans les mêmes conditions opératoires par 2 litres d'eau distillée et opérer comme précédemment, - après détermination de la teneur en matière sèche, ajouter en fonction des extraits aqueux obtenus, un adjuvant de type maltodextrine de manière à obtenir 2/3 d'adjuvant pour 1/3 de matière sèche extraite, - à déshydrater la solution obtenue par atomisation ou par tout autre technique connue de l'homme du métier (lyophilisation, séchage en étuve etc...). Le rendement total en extrait sec de ce procédé est de 15 % en poids par rapport aux écorces.

Dans la composition de l'invention, les inventeurs proposent plus précisément d'utiliser l'ingrédient Eperuline^{™} PW LS 9627, vendu par BASF, et obtenu à partir d'un extrait aqueux d'écorce de l'arbre *Eperua falcata Aubl.* Cet ingrédient, une fois transformé en poudre par pulvérisation, contient 90% de maltodextrine (CAS 9050-36-6), et 10% d'extrait *d'eperua.* Cet ingrédient, qui contient des dihydroflavonols et des tannins catéchiques, empêche la formation de ROS, bloque l'inflammation neurogénique, et améliore la fermeté de la peau.

La composition neurocosmétique de l'invention contient entre 0 et 0,5% en poids d'un extrait d'écorce *d'Eperua falcata,* de préférence entre 0 et 0,3% en poids d'un extrait d'écorce *d'Eperua falcata,* de manière encore plus préférée entre 0 et 0,1% en poids d'un extrait d'écorce *d'Eperua falcata.*

La composition neurocosmétique de l'invention contient par exemple entre 0 et 5% en poids de l'Eperuline^{™} PW LS 9627, de préférence entre 0 et 3% en poids de l'Eperuline^{™} PW LS 9627, de manière encore plus préférée entre 0 et 1 % en poids de l'Eperuline^{™} PW LS 9627.

La composition neurocosmétique de l'invention est de préférence caractérisée en ce qu'elle contient :
a) entre 0.01 et 1% en poids de farine de fruit de *Cocos nucifera,* de préférence de la Sensorialine^{™},
b) entre 0.1 et 0.5% en poids d'un extrait de fleurs, de tiges et/ou de feuilles de *Scutellaria alpina,* de préférence de l'Alpaflor^{®} SCUTELLARIA CB,
et optionnellement :
c) entre 0 et 0.5% en poids d'un extrait d'écorce *d'Eperua falcata,* de préférence de l'Eperuline^{™}.

### Formulation

Les compositions topiques de l'invention peuvent notamment se présenter sous la forme d'une solution aqueuse, hydro-alcoolique ou huileuse, d'une émulsion huile-dans-eau, eau-dans-huile ou d'une émulsion multiple. Elles peuvent également se présenter sous forme de suspensions ou de poudres adaptées à une application sur la peau ou sur les muqueuses.

Les compositions topiques de l'invention peuvent être plus ou moins fluides et avoir l'aspect d'une crème, d'une lotion, d'un lait, d'un sérum, d'une pommade, d'un gel, d'une pâte ou d'une mousse. Elles peuvent également se présenter sous forme solide comme un stick ou être appliquées sur la peau sous forme d'aérosol.

Ainsi, les compositions selon l'invention peuvent être généralement préparées selon une formulation choisie dans le groupe suivant : lotion pour la peau, adoucissant pour la peau, tonique pour la peau, lotion astringente, lotion lactée, lotion hydratante, lotion nourrissante, crème de massage, crème nourrissante, crème hydratante, crème pour les mains, crème anti-âge, crème antirides, produits de maquillage, base de teint, fond de teint, essence, soin enveloppant, mousse nettoyante, shampoing, soin pour le cuir chevelu, soin capillaire, lotion nettoyante, crème nettoyante, lotion pour le corps, nettoyant pour le corps, complément alimentaire, etc.

La composition neurocosmétique de l'invention contient, outre les extraits de plante, de préférence au moins un excipient dermatologiquement acceptable. Cet excipient peut être, par exemple, un excipient utilisé habituellement dans les compositions topiques destinées au soin de la peau.

Dans le contexte de la présente invention, le terme "excipient dermatologiquement acceptable" signifie que ledit excipient convient à une utilisation en contact avec la peau humaine sans toxicité, incompatibilité, instabilité, irritabilité, réponse allergique, etc. Les excipients appropriés comprennent, par exemple, les huiles minérales et les agents émulsifiants. Dans son mode de réalisation le plus simple, le support peut être de l'eau, de l'alcool ou des combinaisons eau / alcool, ou d'autres solvants ou systèmes de solvants dans lesquels les principes actifs susmentionnés peuvent être, par exemple, solubles, dispersés, émulsionnés, etc. La composition de l'invention comprend, de préférence, un ou plusieurs excipients qui permet à la composition d'être uniformément répartie sur la peau et / ou fournissent du corps et de la viscosité à la composition de l'invention, de sorte que les principes actifs pénètrent bien dans la peau, une fois la composition appliquée. Typiquement, l'excipient constitue entre environ 30 et environ 99% en poids de la composition neurocosmétique de l'invention. Des excipients particuliers sont décrits en détail dans, par exemple, US 7 186 404; US 7 175 834; US 7 172 754; US 7 175 835; US 7 101 536; et US 7 078 022. L'homme du métier reconnaîtra et appréciera facilement quels excipients peuvent être utilisés en fonction de la forme et/ou du mode d'administration de la composition de l'invention.

Lorsque la composition neurocosmétique selon l'invention est formulée sous forme de pâte, de crème ou de gel, une fibre animale, une fibre végétale, une cire, de la paraffine, de l'amidon, de la gomme adragante, un dérivé cellulosique, du polyéthylène glycol, de la silicone, de la bentonite, de la silice, du talc, de l'oxyde de zinc, etc. peuvent être utilisés comme composant de support.

Lorsque la composition neurocosmétique selon la présente invention est formulée sous forme de poudre ou de spray, du lactose, du talc, de la silice, de l'hydroxyde d'aluminium, du silicate de calcium ou de la poudre de polyamide peuvent être utilisés comme composant support. En particulier, lorsqu'elle est formulée sous forme de spray, elle peut comprendre en outre un agent propulseur tel que le chlorofluorohydrocarbure, le propane/butane ou le diméthyléther.

Lorsque la composition neurocosmétique selon la présente invention est formulée sous forme de solution ou d'émulsion, un solvant, un solubilisant ou un émulsifiant peut être utilisé comme composant support. Par exemple, l'eau, l'éthanol, l'isopropanol, le carbonate d'éthyle, l'acétate d'éthyle, l'alcool benzylique, le benzoate de benzyle, le propylène glycol, l'huile de 1,3-butyl glycol, un ester gras de glycérol ou un ester d'acide gras de polyéthylène glycol ou de sorbitan peuvent être utilisés.

Lorsque la composition neurocosmétique selon l'invention est formulée sous forme de suspension, un diluant tel que l'eau, l'éthanol ou le propylène glycol, un agent de suspension tel que l'alcool isostéarylique éthoxylé, le polyoxyéthylène sorbitol ester et le polyoxyéthylène sorbitan ester, la cellulose microcristalline, le métahydroxyde d'aluminium, la bentonite, de la gélose, de la gomme adragante, etc. peuvent être utilisés comme composant support.

Lorsque la composition neurocosmétique selon l'invention est formulée sous la forme d'un agent nettoyant, un tensioactif, un sulfate d'alcool gras, un sulfate d'éther d'alcool gras, un monoester d'acide sulfosuccinique, de l'iséthionate, un dérivé d'imidazolinium, du taurate de méthyle, du sarcosinate, un sulfate d'éther d'amide d'acide gras, un une alkylamidobétaïne, un alcool gras, un glycéride d'acide gras, un diéthanolamide d'acide gras, une huile végétale, un dérivé de lanoline, ou un ester d'acide gras de glycérol éthoxylé, peuvent être utilisés comme composant support.

La composition neurocosmétique selon l'invention peut en outre comprendre un additif fonctionnel et des ingrédients couramment utilisés dans une composition neurocosmétique, en plus des extraits de plante mentionnés ci-dessus. Cet additif fonctionnel peut être choisi dans le groupe constitué par : une vitamine hydrosoluble, une vitamine soluble dans l'huile, un polypeptide, un polysaccharide, un sphingolipide et un extrait d'algue.

Le cas échéant, la composition neurocosmétique de l'invention peut en outre comprendre, en plus de l'additif fonctionnel, les ingrédients couramment utilisés dans une composition neurocosmétique.

Des exemples d'autres ingrédients compris peuvent comprendre une huile, une graisse, un humectant, un émollient, un tensioactif, un pigment organique ou inorganique, une poudre organique, un absorbant UV, un conservateur, un stérilisant, un antioxydant, un extrait végétal, un agent de contrôle du pH, un alcool, un colorant, un arôme, un promoteur de circulation sanguine, un agent rafraîchissant, un anti-transpirant, de l'eau purifiée, etc.

La composition neurocosmétique de l'invention peut être formulée de préférence sous forme de gel, d'huile ou de lotion, pour une application topique, sur la peau.

De préférence, l'émulsion de l'invention contient, comme excipient, de l'eau, des solvants organiques miscibles à l'eau (comme les alcools), des agents gélifiants/stabilisants (tels que des gommes, des polymères synthétiques et leurs dérivés), des corps gras liquides (tels que les huiles volatiles ou non volatiles), des corps gras solides tels que les cires, les corps gras pâteux, les gommes et leurs mélanges, des agents tensioactifs émulsionnants parmi des agents tensioactifs anioniques, cationiques, amphotères ou non ioniques, une phase particulaire additionnelle pouvant notamment comprendre au moins un pigment et/ou au moins une nacre et/ou au moins une charge complémentaire, des colorants hydrosolubles ou liposolubles, des parfums ou bien des agents alcalinisants ou acidifiants, des agents antioxydants, ou des conservateurs ou un autre excipient connu, ou leur mélange.

Dans tous les cas, l'homme du métier veillera à ce que lesdits excipients ainsi que leurs proportions soient choisis de manière à ne pas interférer avec les propriétés avantageuses recherchées de la composition de l'invention. Ces excipients peuvent par exemple correspondre à 0,01 à 20% du poids total de la composition. Lorsque la composition de l'invention est une émulsion, la phase grasse peut représenter 5 à 80% en poids et de préférence 5 à 50% en poids par rapport au poids total de la composition. Les émulsionnants et co-émulsionnants utilisés dans la composition seront choisis parmi ceux classiquement utilisés dans le domaine considéré. Par exemple, ils peuvent être utilisés dans une proportion allant de 0,3 à 30% en poids, par rapport au poids total de la composition.

Avantageusement, la composition de l'invention peut comprendre, en plus des extraits de plante de l'invention, au moins un autre agent actif ayant des effets cosmétiques similaires et / ou complémentaires à ceux de l'invention. Selon l'invention, cet agent actif sera défini comme un "agent actif supplémentaire".

Par exemple, ledit agent actif supplémentaire peut être choisi parmi les agents anti-âge, tonifiants, éclaircissants, hydratants, drainants, agents favorisant la microcirculation, exfoliants, gommants, agents stimulant la matrice extracellulaire, agents activant le métabolisme énergétique, agents antibactériens, antifongiques, calmants, anti-radicaux libres, anti-UV et anti-acnéiques, anti-inflammatoires, anesthésiques, réchauffants, rafraîchissants et autres agents amincissants.

Plus particulièrement, cet agent actif peut être par exemple choisi parmi : les agents antiglycation et/ou antioxydants pris seuls ou en association (tels que par exemple le tocophérol et ses dérivés, l'ergothionéine, la thiotaurine, l'hypotaurine, l'aminoguanidine, la thiamine pyrophosphate, la pyridoxamine, la lysine, l'histidine, l'arginine, la phénylalanine, la pyridoxine, l'adénosine triphosphate), les agents anti-inflammatoires (tels que par exemple l'alpha-bisabolol ou le stearyl glycyrrhetinate), les agents apaisants et leurs mélanges, les agents conservateurs (anti-bactériens ou anti-fongiques), les agents hydratants, les modificateurs de pH, les agents kératolytiques et/ou desquamants (tels que par exemple l'acide salicylique et ses dérivés), les vitamines, les filtres solaires, les agents autobronzants les acides gras essentiels, les absorbeurs d'odeurs, ou encore tout additif couramment utilisé en cosmétique, ainsi que tous les adjuvants nécessaires à leur formulation, tels que solvants, épaississants, diluants, colorants, pigments, charges, conservateurs, parfums, huiles essentielles, vitamines, tensioactifs, polymères filmogènes, etc.

Il est notamment possible d'ajouter, dans la composition de l'invention, un autre agent neurocosmétique connu pour stimuler la sécrétion d'endorphine, comme par exemple du peptide NATAH, de la calmosensine^{™}, une des molécules endorphine-like décrites dans les demandes EP1498113, FR2857588 et EP 1 299 081, et notamment du D-stachyose, du cicéritol, ou un extrait de *Tephrosia purpurea* (cf. EP 1 299 081), le dermilyn, la caobromine, le stoechiol, le calmiskin, la clotholine, etc.

Le terme « physiologiquement acceptable » fait référence ici à tout composé adapté pour entrer en contact avec la peau ou une muqueuse sans provoquer de réactions de toxicité ou d'intolérance.

En règle générale, les compositions neurocosmétiques de l'invention peuvent être administrées une à deux fois par jour, tous les jours. Les variations de ces niveaux de dosage peuvent être ajustées en utilisant des routines empiriques standard pour l'optimisation, comme cela est bien compris dans l'art. Des indications quant aux dosages particuliers et aux méthodes d'administration sont fournies dans la littérature et sont généralement disponibles pour les praticiens de l'art. L'homme du métier emploiera différentes formulations en fonction de la nature, par exemple, de la structure, de la composition, du constituant limonoïde actif.

### Utilisations

L'invention a également pour objet un procédé de traitement cosmétique et/ou l'utilisation de la composition de l'invention, pour maintenir ou favoriser la luminosité et le teint de la peau, renforcer la fonction barrière de la peau, et prévenir les effets du stress sur la peau, et favoriser la résistance de la peau aux agressions externes liées à l'environnement ou aux modifications provoquées par des stress intrinsèques, ledit procédé comprenant l'application d'une quantité efficace de la composition de l'invention, telle que décrite ci-dessous. Cette composition pourra être appliquée sur la partie de la peau à traiter, en particulier sur le visage, le corps, le cou ou les mains, de manière quotidienne ou pluriquotidienne. Avantageusement, elle procure un effet anti-âge, antiride, elle renforce la fermeté et l'éclat de la peau, et elle hydrate la peau.

Plus particulièrement, la présente invention vise l'utilisation de la composition décrite ci-dessus pour augmenter la survie des neurones sensoriels cutanés, pour augmenter la pousse neuritique des neurones cutanés, ou pour augmenter l'expression des récepteurs µ-opioïdes tels que les récepteurs de la beta-endorphine.

Ces effets avantageux ont été démontrés par les inventeurs pour des compositions contenant de la noix de coco et de la Scutellaire des Alpes (« Mix 4 », figures 1-20). Certains sont encore plus marqués lorsque la composition contient en outre un extrait *d'Eperua falcata* (« Mix 5 », figures 1-20). Pour la plupart de ces effets, la composition de l'invention (contenant les deux ou trois extraits de plantes de l'invention) agit, de manière surprenante, plus efficacement sur les neurones que la bêta-endorphine elle-même (cf. exemples ci-dessous).

La composition neurocosmétique de l'invention est préférentiellement appliquée sur des peaux soumises à un stress aigu ou chronique. Elle peut aussi être appliquée sur la peau en prévention d'un tel stress, ou en l'absence de stress.

Le stress cutané peut être induit par différents facteurs de stress. Il peut résulter de la présence d'agents polluants (ozone, métaux lourds, radicaux libres), ou de variations brusques de la température ou de l'humidité relative de l'atmosphère, dues au vent, à l'air conditionné, ou aux contraintes mécaniques. Il peut s'agir d'un stress mécanique, une irritation provoquée par certains tissus rugueux ou soins d'hygiène trop agressifs, ou encore un stress psychologique dû à la fatigue, à l'anxiété, etc. Tous ces facteurs ayant un effet néfaste sur l'état de la peau.

Plus précisément, la composition de l'invention peut être utilisée pour prévenir les effets délétères d'un stress aigu ou chronique sur la peau et/ou pour renforcer la résistance de la peau aux stress chroniques ou aigus.

Par son effet « endorphine-like », elle peut également être utilisée pour améliorer l'homéostasie et/ou pour apporter une sensation de bien-être, notamment pour diminuer dans les neurones de la peau l'expression des récepteurs aux glucocorticoïdes, tels que les récepteurs du cortisol, notamment en présence d'un stress chronique.

La composition de l'invention peut plus généralement être utilisée pour améliorer l'hydratation de la peau et/ou améliorer la fonction barrière de la peau et/ou améliorer l'éclat de la peau. Après application, la peau est plus lumineuse, plus douce au toucher.

La composition neurocosmétique de l'invention peut enfin procurer à ses utilisateurs une sensation agréable et un bien-être psychologique lié à l'effet de stimulation des neurones cutanés. En particulier, elle peut procurer un sentiment de repos, de relaxation, et/ou d'apaisement, une sensation de rééquilibrage ou d'équilibre.

### Définitions

Par «extrait de plante», on entend toute substance ou préparation isolée extraite d'une plante, indépendamment de la méthode d'extraction utilisée. Le terme est utilisé au sens large, comprenant, par exemple, des ingrédients solubles dans l'eau ou des ingrédients organiques extraits à l'aide du solvant, ou des ingrédients spécifiques de la plante. Le terme comprend également toute substance pouvant être obtenue par transformation ou traitement d'une substance extraite d'un produit naturel. Spécifiquement, ladite transformation ou ledit traitement peut être une fermentation ou un traitement enzymatique de l'extrait.

Tel qu'utilisé dans la présente demande, le terme « % » fait référence au « % en poids », c'est-à-dire au pourcentage en poids d'un composant par rapport au poids total de la composition (c'est-à-dire, y compris tous supports, véhicules, solvants, charges ou autres composants ajoutés avant l'application sur la peau), sauf disposition contraire.

### LEGENDES DES FIGURES

**Figure 1****:** Dosage par ELISA de la quantité de neuropeptide CGRP libérée par la co-culture traitée pendant 10 jours par les mélanges d'actifs à 3 concentrations ou la β-endorphine. Quantité de CGRP normalisée par la condition témoin traité pardu milieu contrôle (Témoin ; 100%). Moyennes de 6 réplicats +/- s.e.m. Statistiques réalisées à l'aide d'un test One Way ANOVA avec correction de Dunnett avec le témoin traité par du milieu contrôle comme référence (**** p<0.0001).
**Figure 2****:** Dosage par ELISA de la quantité de neuropeptide CGRP libérée par la co-culture traitée pendant 10 jours par les mélanges d'actifs à 3 concentrations ou la β-endorphine et après 30 minutes d'activation par la capsaïcine. Quantité de CGRP normalisée par la condition témoin traité par du milieu contrôle (100%). Moyennes de 6 réplicats +/- s.e.m. Statistiques réalisées à l'aide d'un test One Way ANOVA avec correction de Dunnett avec le témoin capsaïcine comme référence (**** p<0.0001).

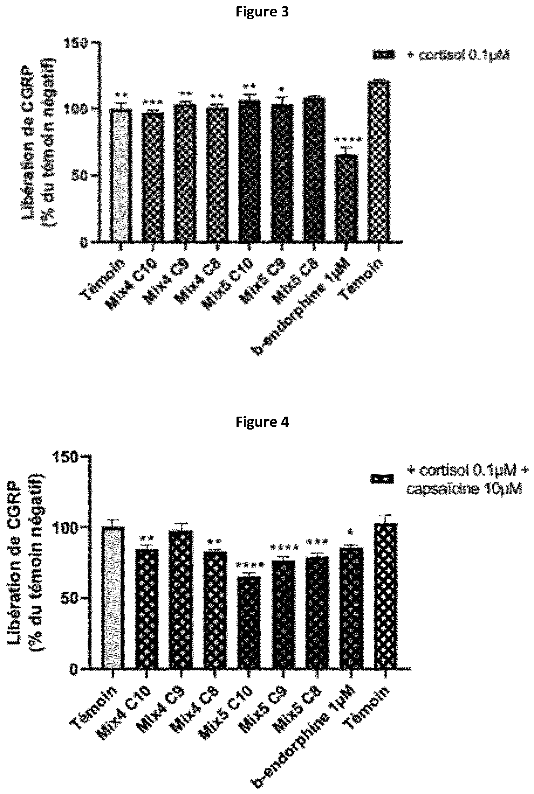

**Figure 3** : Dosage par ELISA de la quantité de neuropeptide CGRP libérée par la co-culture traitée pendant 10 jours par les mélanges d'actifs à 3 concentrations ou la β-endorphine et en présence de cortisol. Quantité de CGRP normalisée par la condition témoin traité par du milieu contrôle (100%). Moyennes de 6 réplicats +/- s.e.m. Statistiques réalisées à l'aide d'un test One Way ANOVA avec correction de Dunnett avec le témoin cortisol comme référence (* p<0.05 ; ** p<0.01 ; **** p<0.0001).
**Figure 4** : Dosage par ELISA de la quantité de neuropeptide CGRP libérée par la co-culture traitée pendant 10 jours par les mélanges d'actifs à 3 concentrations ou la β-endorphine et en présence de cortisol et activée 30 minutes avant fixation par de la capsaïcine. Quantité de CGRP normalisée par la condition témoin traité par du milieu contrôle (100%). Moyennes de 6 réplicats +/- s.e.m. Statistiques réalisées à l'aide d'un test One Way ANOVA avec correction de Dunnett avec le témoin cortisol comme référence (* p<0.05 ; *** p<0.001 : **** p<0.0001).

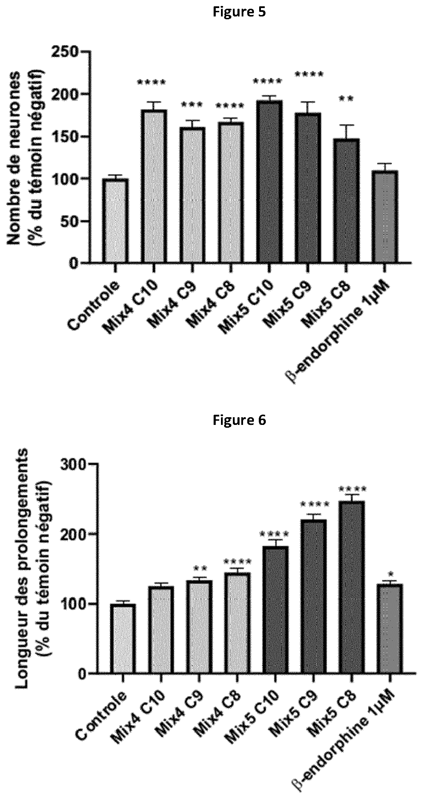

**Figure 5** : Dénombrement des neurones sensitifs humains dans la co-culture traitée pendant 10 jours par les mélanges d'actifs à 3 concentrations ou la β-endorphine. Nombre de neurones sensitifs normalisé par le témoin traité par du milieu contrôle (100%). Moyennes de 6 réplicats +/- s.e.m. Les statistiques présentées sur le graphique ont été réalisées à l'aide du test One Way ANOVA par rapport au témoin traité par du milieu contrôle (** p<0.01 ; *** p<0.001 ; **** p<0.0001).
**Figure 6** : Evaluation de la longueur des prolongements des neurones sensitifs humains dans la co-culture traitée pendant 10 jours par les mélanges d'actifs à 3 concentrations ou la β-endorphine. La longueur des prolongements a été rapportée au nombre de neurones puis normalisée par la condition témoin traité du milieu contrôle (100%). Moyennes de 6 réplicats +/- s.e.m. Les statistiques présentées sur le graphique ont été réalisées à l'aide du test One Way ANOVA par rapport au témoin traité par du milieu contrôle. (* p<0.05; ** p<0.01 ; **** p<0.0001).

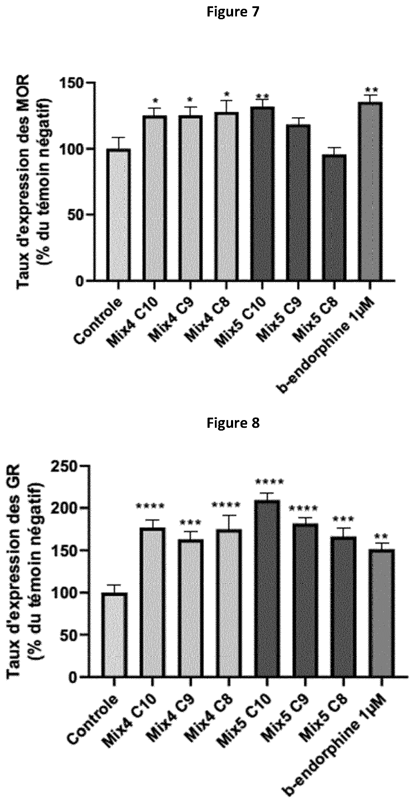

**Figure 7** : Evaluation du taux d'expression des MOR par les neurones sensitifs humains dans la co-culture traitée pendant 10 jours par les mélanges d'actifs à 3 concentrations ou la β-endorphine. Le taux d'expression a été normalisé par la condition témoin traité par du milieu contrôle (100%). Moyennes de 6 réplicats +/- s.e.m. Les statistiques présentées sur le graphique ont été réalisées à l'aide du test One Way ANOVA par rapport au témoin traité par du milieu contrôle. (* p<0.05 ; ** p<0.01).
**Figure 8** : Evaluation du taux d'expression des GR par les neurones sensitifs humains dans la co-culture traitée pendant 10 jours par les mélanges d'actifs à 3 concentrations ou la β-endorphine. Le taux d'expression des récepteurs GR a été normalisé par la condition témoin traité par du milieu contrôle (100%). Moyennes de 6 réplicats +/- s.e.m. Les statistiques présentées sur le graphique ont été réalisées à l'aide du test One Way ANOVA par rapport au témoin traité par du milieu contrôle. (** p<0.01 ; *** p<0.001 ; **** p<0.0001).

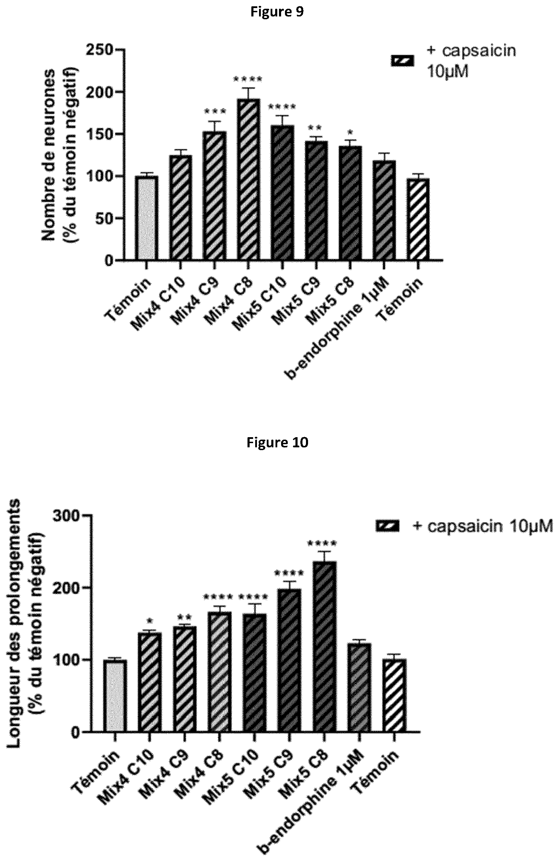

**Figure 9** : Dénombrement des neurones sensitifs humains dans la co-culture traitée pendant 10 jours par les mélanges d'actifs à 3 concentrations ou la β-endorphine et activée 30 minutes par la capsaïcine à 10µM en fin de culture. Le nombre de neurones sensitif a été normalisé par la condition témoin traité par du milieu contrôle (100%). Moyennes +/- s.e.m. Les statistiques présentées sur le graphique ont été réalisées à l'aide du test One Way ANOVA par rapport au témoin capsaïcine (* p<0.05; ** p<0.01 ; *** p<0.001 ; **** p<0.0001).
**Figure 10** : Evaluation de la longueur des prolongements des neurones sensitifs humains dans la co-culture traitée pendant 10 jours par les mélanges d'actifs à 3 concentrations ou la molécule de référence et activée 30 minutes par la capsaïcine à 10µM en fin de culture. La longueur des prolongements a été rapportée au nombre de neurones puis normalisée par le témoin traité par du milieu contrôle (100%). Moyennes de 6 réplicats +/- s.e.m. Les statistiques présentées sur le graphique ont été réalisées à l'aide du test One Way ANOVA par rapport au témoin capsaïcine. (* p<0.05 ; ** p<0.01; **** p<0.0001).

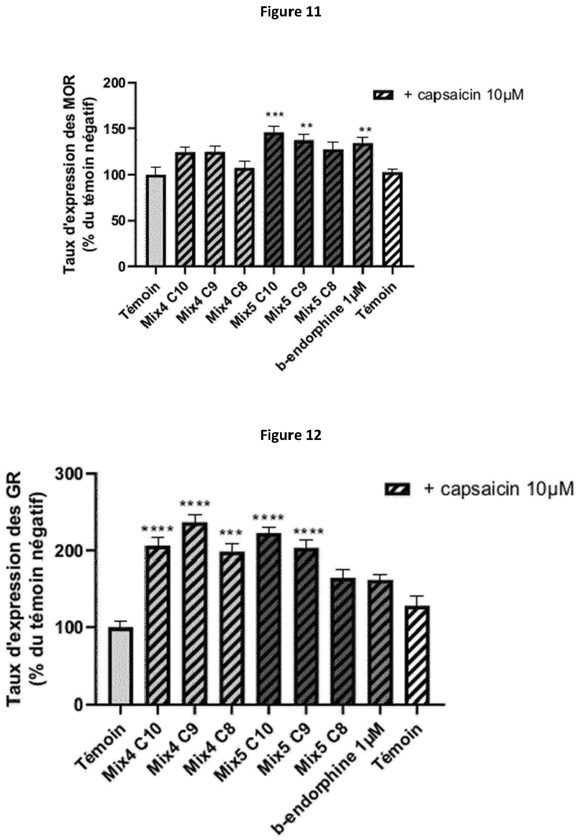

**Figure 11** : Evaluation du taux d'expression des µ-opioïdes récepteurs (MOR) par les neurones sensitifs humains dans la co-culture traitée pendant 10 jours par les mélanges d'actifs à 3 concentrations ou la β-endorphine et activée 30 minutes par la capsaïcine à 10µM en fin de culture. Le taux d'expression des MOR a été normalisé par le témoin traité par du milieu contrôle (100%). Moyennes de 6 réplicats +/- s.e.m. Les statistiques présentées sur le graphique ont été réalisées à l'aide du test One Way ANOVA par rapport au témoin capsaïcine. (** p<0.1, *** p<0.001).
**Figure 12** : Evaluation du taux d'expression des récepteurs aux glucocorticoïdes (GR) par les neurones sensitifs humains dans la co-culture traitée pendant 10 jours par les mélanges d'actifs à 3 concentrations ou la β-endorphine et activée 30 minutes par la capsaïcine à 10µM en fin de culture. Le taux d'expression des GR a été normalisé par le témoin traité par du milieu contrôle (100%). Moyennes de 6 réplicats +/- s.e.m. Les statistiques présentées sur le graphique ont été réalisées à l'aide du test One Way ANOVA par rapport au témoin capsaïcine (*** p<0.001 ; **** p<0.0001).

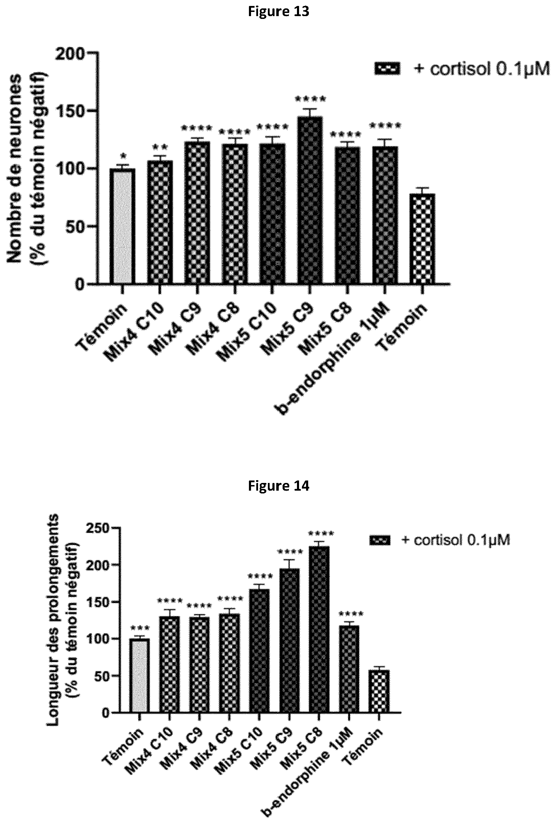

**Figure 13** : Dénombrement des neurones sensitifs humains dans la co-culture traitée pendant 10 jours du cortisol à 0.1µM en présence des mélanges d'actifs à 3 concentrations ou de la β-endorphine. Le nombre de neurones sensitifs a été normalisé par le témoin traité par du milieu contrôle (100%). Moyennes de 6 réplicats +/- s.e.m. Les statistiques présentées sur le graphique ont été réalisées à l'aide du test One Way ANOVA par rapport au témoin cortisol (* p<0.05;** p<0.01 ; **** p<0.0001).
**Figure 14** **:** Evaluation de la longueur des prolongements des neurones sensitifs humains dans la co-culture traitée pendant 10 jours du cortisol à 0.1µM en présence des mélanges d'actifs à 3 concentrations ou de la β-endorphine. La longueur des prolongements a été rapportée au nombre de neurones puis normalisée par le témoin traité par du milieu contrôle (100%). Moyennes de 6 réplicats +/- s.e.m. Les statistiques présentées sur le graphique ont été réalisées à l'aide du test One Way ANOVA par rapport au témoin cortisol. (*** p<0.001 ; **** p<0.0001).

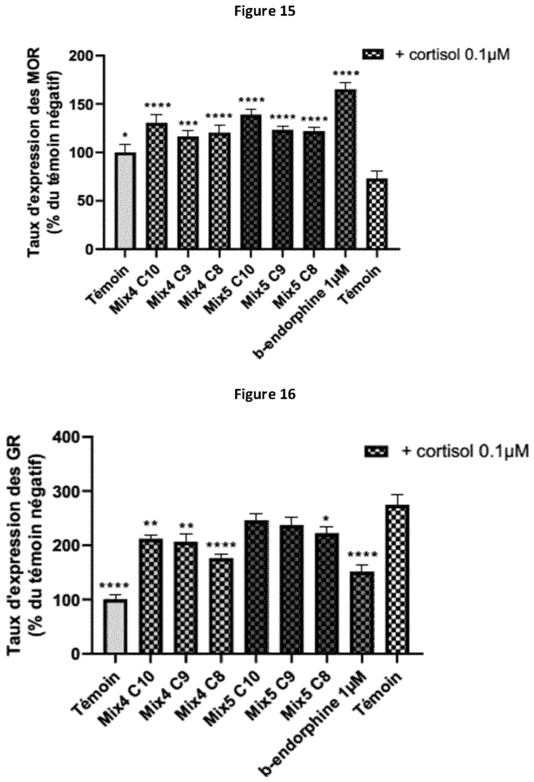

**Figure 15** : Evaluation du taux d'expression des µ-opioïdes récepteurs (MOR) par les neurones sensitifs humains dans la co-culture traitée pendant 10 jours du cortisol à 0.1µM en présence des mélanges d'actifs à 3 concentrations ou de la β-endorphine. Le taux d'expression des MOR a été normalisé par le témoin traité par du milieu contrôle (100%). Moyennes de 6 réplicats +/- s.e.m. Les statistiques présentées sur le graphique ont été réalisées à l'aide du test One Way ANOVA par rapport au témoin cortisol. (* p<0.05 ; *** p<0.001; **** p<0.0001).
**Figure 16** : Evaluation du taux d'expression des récepteurs aux glucocorticoïdes (GR) par les neurones sensitifs humains dans la co-culture traitée pendant 10 jours du cortisol à 0.1µM en présence des mélanges d'actifs à 3 concentrations ou de la β-endorphine. Le taux d'expression des GR a été normalisé par le témoin traité par du milieu contrôle (100%) Moyennes de 6 réplicats +/- s.e.m. Les statistiques présentées sur le graphique ont été réalisées à l'aide du test One Way ANOVA par rapport au témoin cortisol. (* p<0.05 ; ** p<0.01 ; **** p<0.0001).

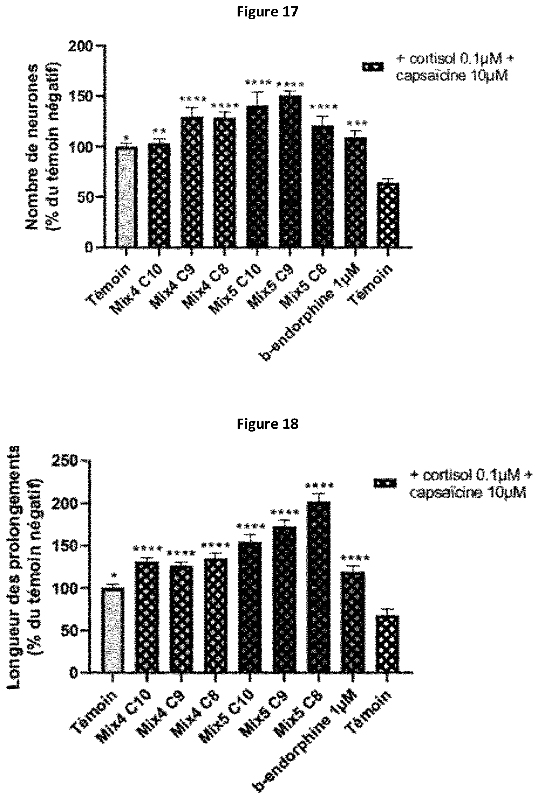

**Figure 17** : Dénombrement des neurones sensitifs humains dans la co-culture traitée pendant 10 jours du cortisol à 0.1µM en présence des mélanges d'actifs à 3 concentrations ou de la β-endorphine et activée 30 minutes par la capsaïcine à 10µM en fin de culture. Le nombre de neurones a été normalisé par le témoin traité par du milieu contrôle (100%). Moyennes de 6 réplicats +/- s.e.m. Les statistiques présentées sur le graphique ont été réalisées à l'aide du test One Way ANOVA par rapport au témoin cortisol + capsaïcine (* p<0.05 ; ** p<0.01; *** p<0.001 ; **** p<0.0001).
**Figure 18** : Evaluation de la longueur des prolongements des neurones sensitifs humains dans la co-culture traitée pendant 10 jours du cortisol à 0.1µM en présence des mélanges d'actifs à 3 concentrations ou de la β-endorphine et activée 30 minutes par la capsaïcine à 10µM en fin de culture. La longueur des prolongements a été rapportée au nombre de neurones puis normalisée par le témoin traité par du milieu contrôle (100%). Moyennes de 6 réplicats +/- s.e.m. Les statistiques présentées sur le graphique ont été réalisées à l'aide du test One Way ANOVA par rapport au témoin cortisol + capsaïcine. (* p<0.05 ; **** p<0.0001).

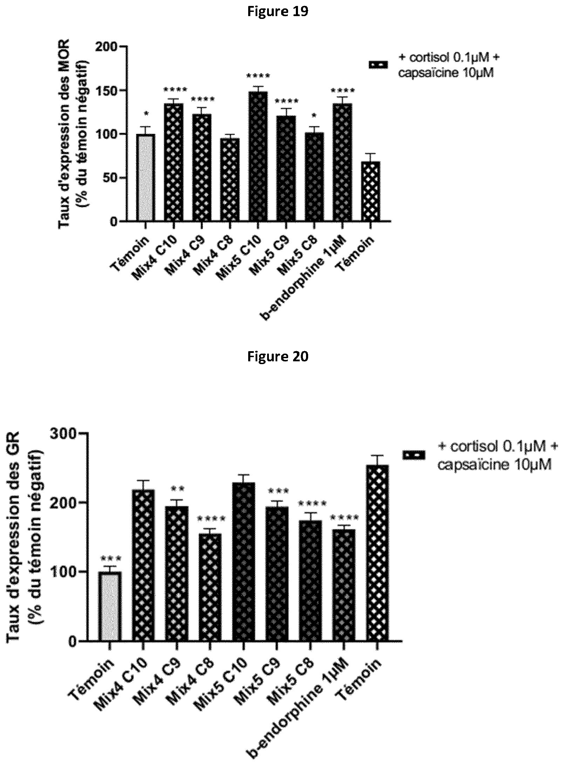

**Figure 19** : Evaluation du taux d'expression des µ-opioïdes récepteurs (MOR) par les neurones sensitifs humains dans la co-culture traitée pendant 10 jours du cortisol à 0.1µM en présence des mélanges d'actifs à 3 concentrations ou de la β-endorphine et activée 30 minutes par la capsaïcine à 10µM en fin de culture. Le taux d'expression des MOR a été normalisé par la condition témoin traité par du milieu contrôle (100%). Moyennes de 6 réplicats +/- s.e.m. Les statistiques présentées sur le graphique ont été réalisées à l'aide du test One Way ANOVA par rapport au témoin cortisol + capsaïcine (* p<0.05 ; **** p<0.0001).
**Figure 20** : Evaluation du taux d'expression des récepteurs aux glucocorticoïdes (GR) par les neurones sensitifs humains dans la co-culture traitée pendant 10 jours du cortisol à 0.1µM en présence des mélanges d'actifs à 3 concentrations ou de la β-endorphine et activée 30 minutes par la capsaïcine à 10µM en fin de culture. Le taux d'expression des GR a été normalisé par le témoin traité par du milieu contrôle (100%). Moyennes de 6 réplicats +/- s.e.m. Les statistiques présentées sur le graphique ont été réalisées à l'aide du test One Way ANOVA par rapport au témoin cortisol + capsaïcine. (** p<0.01 ; *** p<0.001 ; **** p<0.0001).

### EXEMPLES

### Matériel et méthodes

### 1.1. Co-culture des neurones sensitifs humains et des kératinocytes

Les neurones sensitifs sont dérivés de cellules hiPS (human induced Pluripotent Stem cells) elles-mêmes obtenues à partir de fibroblastes humains. Les cellules ont été ensemencées en plaques 96 puits et maintenues 6 jours en culture, dans un milieu induisant leur différenciation, à 37°C et 5% de COz. Le milieu de culture a été changé tous les 2 jours.

Après 9 jours de culture, le milieu M1 a été changé par un milieu de maturation pour neurones sensitifs. Les cellules ont été maintenues en culture à 37°C et 5% de COz. Le milieu a été changé tous les 2 à 3 jours.

Après 14 jours de culture, les kératinocytes commerciaux issus d'un donneur adulte de 29 ans (PromoCell; ref: C12013) ont été ajoutés dans les plaques au-dessus du tapis d'hiPS différenciés. La co-culture a été maintenue dans milieu de culture constitué d'un mix de milieu M2 et de milieu de croissance pour kératinocytes (Promocell ; ref : C-20111) à 37°C et 5% de COz et contenant du cortisol à 0.1µM et/ou les actifs. Le milieu de culture a été changé tous les 2 à 3 jours avec les différents produits.

### 1.2. Traitement de la co-culture par les actifs et le cortisol

Après 9 jours de cultures les mélanges d'actifs, le cortisol et la β-endorphine ont été ajoutés sur les co-cultures selon les conditions suivantes.

Le mélange appelé « Mix4 » contient :

| **MIX 4** | | **C8** | **C9** | **C10** |
|---|---|---|---|---|
| Alpaflore Scutellaria (ALPAFLORE^{™}) | | 0.114% | 0,04% | 0.02% |
| Sensorialine | | 0,0275% | 0,0275% | 0,01375% |

Le mélange appelé « Mix5 » contient :

| **MIX 5** | | **C8** | **C9** | **C10** |
|---|---|---|---|---|
| Alpaflore Scutellaria (ALPAFLORE^{™}) | | 0.114% | 0,04% | 0.02% |
| Eperuline^{™} PW LS 9627 | | 0,006% | 0,006% | 0,003% |
| Sensorialine | | 0,0275% | 0,0275% | 0,01375% |

- Milieu contrôle (témoin non traité)
- Milieu contrôle + Mix4 C8
- Milieu contrôle + Mix4 C9
- Milieu contrôle + Mix4 C10
- Milieu contrôle + Mix5 C8
- Milieu contrôle + Mix5 C9
- Milieu contrôle + Mix5 C10
- Milieu contrôle + Mix4 C8 + cortisol à 0.1µM
- Milieu contrôle + Mix4 C9 + cortisol à 0.1µM
- Milieu contrôle + Mix4 C10 + cortisol à 0.1µM
- Milieu contrôle + Mix5 C8 + cortisol à 0.1µM
- Milieu contrôle + Mix5 C9 + cortisol à 0.1µM
- Milieu contrôle + Mix5 C10 + cortisol à 0.1µM
- Milieu contrôle + β-endorphine à 1µM
- Milieu contrôle + β-endorphine à 1µM + cortisol à 0.1µM
- Milieu contrôle + cortisol à 0.1µM

16 conditions de culture ont été réalisées, n=12 puits. Les milieux de culture ont été changés tous les 2 ou 3 jours.

Après 19 jours de culture (soit 10 jours d'incubation des composés), les milieux ont été prélevés et stockés à -80°C. Les cellules ont alors été activées par de la capsaïcine à 10µM. Les conditions suivantes ont été réalisées :
- Milieu contrôle
- Milieu contrôle + capsaïcine 10µM
- Milieu contrôle + Mix4 C8
- Milieu contrôle + Mix4 C9
- Milieu contrôle + Mix4 C10
- Milieu contrôle + Mix5 C8
- Milieu contrôle + Mix5 C9
- Milieu contrôle + Mix5 C10
- Milieu contrôle + Mix4 C8 + capsaïcine 10µM
- Milieu contrôle + Mix4 C9 + capsaïcine 10µM
- Milieu contrôle + Mix4 C10 + capsaïcine 10µM
- Milieu contrôle + Mix5 C8 + capsaïcine 10µM
- Milieu contrôle + Mix5 C9 + capsaïcine 10µM
- Milieu contrôle + Mix5 C10 + capsaïcine 10µM
- Milieu contrôle + Mix4 C8 + cortisol à 0.1µM
- Milieu contrôle + Mix4 C9 + cortisol à 0.1µM
- Milieu contrôle + Mix4 C10 + cortisol à 0.1µM
- Milieu contrôle + Mix5 C8 + cortisol à 0.1µM
- Milieu contrôle + Mix5 C9 + cortisol à 0.1µM
- Milieu contrôle + Mix5 C10 + cortisol à 0.1µM
- Milieu contrôle + Mix4 C8 + cortisol 0.1µM + capsaïcine 10µM
- Milieu contrôle + Mix4 C9 + cortisol 0.1µM + capsaïcine 10µM
- Milieu contrôle + Mix4 C10 + cortisol 0.1µM+ capsaïcine 10µM
- Milieu contrôle + Mix5 C8 + cortisol 0.1µM + capsaïcine 10µM
- Milieu contrôle + Mix5 C9 + cortisol 0.1µM + capsaïcine 10µM
- Milieu contrôle + Mix5 C10 + cortisol 0.1µM+ capsaïcine 10µM
- Milieu contrôle + β-endorphine à 1µM
- Milieu contrôle + β-endorphine à 1µM + capsaïcine 10µM
- Milieu contrôle + β-endorphine à 1µM + cortisol à 0.1µM
- Milieu contrôle + β-endorphine à 1µM + cortisol à 0.1µM + capsaïcine 10µM
- Milieu contrôle + cortisol à 0.1µM
- Milieu contrôle + cortisol à 0.1µM + capsaïcine 10µM

Après 30 minutes de stimulation, les surnageants de culture ont été récupérés et stockés à -80°C jusqu'au dosage. Les tapis cellulaires ont alors été fixés dans une solution de paraformaldéhyde. 32 conditions de culture ont été réalisées, n=6 puits par condition.

### 1.3. Marquage des neurones et évaluation de l'expression des MOR, des GR, du nombre de neurones sensitifs ainsi que de la longueur des prolongements

Les 32 conditions de traitement des deux cultures ont été analysées.

Les cellules préalablement fixées, ont été incubées en présence d'anticorps primaires anti β-tubuline (Sigma Aldrich), anti-MOR (Biotechne) et anti-GR (Sigma Aldrich). L'anticorps anti β-tubuline marque le corps cellulaire et les prolongements des neurones. L'anticorps anti-MOR marque les récepteurs de la β- endorphine exprimés par les neurones sensitifs et l'anticorps anti-GR les récepteurs aux glucocorticoïdes (récepteurs du cortisol). Ces anticorps ont été révélés par des anticorps secondaires couplés à des fluorochromes (Fisher Scientific). Les noyaux ont été marqués par une solution de Hoechst (Sigma Aldrich), un marqueur fluorescent nucléaire, dans la même solution que les anticorps secondaires.

Par puits de culture, 20 photographies ont été prises avec le microscope automatique (InCell 2200 ; GE Healthcare) au grossissement x20. Un dénombrement des neurones a été effectué, la longueur des prolongements a été mesurée pour chaque condition et les résultats ont été comparés à la condition contrôle non traité. La densité d'expression des marqueurs MOR et GR exprimés par les neurones sensitifs a également été déterminée et comparée à la condition contrôle.

Une analyse statistique a été réalisée à l'aide d'un test One Way Anova suivi d'un test de Dunnett.

Par condition de culture, 6 puits ont été réalisés.

### 1.4. Dosage de la quantité de neuropeptide CGRP libérée

Les surnageants prélevés après 30 minutes d'activation par la capsaïcine ont été décongelés et un dosage de la quantité de neuropeptide CGRP libérée a été réalisé par ELISA (Abbexa ; ref : abx257902). Les quantités de CGRP libérées dans les différentes conditions ont été comparées à celle mesurée dans la condition traitée par la capsaïcine seule. Une analyse statistique a été réalisée à l'aide d'un test One Way Anova suivi d'un test de Dunnett.
32 conditions de culture ont été analysées, n=6 puits

### Résultats

Les résultats de l'étude sont présentés, pour chaque paramètre analysé, sous forme de quatre graphiques correspondant aux quatre séries de conditions de traitement suivantes:
- Basal : Mix ou β-endorphine seuls avec traitement pendant 10 jours
- Cortisol : Mix ou β-endorphine en présence de cortisol avec traitement pendant 10 jours
- Capsaïcine : Mix ou β-endorphine seuls avec traitement pendant 10 jours puis activation 30 minutes par la capsaïcine
- Cortisol + capsaïcine : Mix ou β-endorphine en présence de cortisol avec traitement pendant 10 jours puis activation 30 minutes par la capsaïcine

### 1. Dosage de la quantité de neuropeptide GCRP libérée

### 1.1. Conditions basales

Comme le montre la figure 1, le traitement de la co-culture pendant 10 jours par la β-endorphine à 1µM n'entraine pas de modulation de la libération de neuropeptide CGRP par rapport au témoin traité par du milieu contrôle.

Le traitement de la co-culture pendant 10 jours par le Mix4 aux concentrations C10, C9 ou C8, entraine une augmentation non significative de la libération de neuropeptide CGRP par rapport au témoin traité par du milieu contrôle (respectivement plus 18%, 23% et 24%).

Le traitement de la co-culture pendant 10 jours par le Mix5 aux concentrations C10, C9 et C8 entraine une augmentation significative de la libération de neuropeptide CGRP par rapport au témoin traité par du milieu contrôle (respectivement plus 84% ; p<0.0001, 83% ; p<0.0001 et 106% ; p<0.0001). Cette augmentation en conditions basales est probablement due à l'augmentation de la survie et du nombre des neurones, et non à un effet pro-inflammatoire.

### 1.2. Conditions activées 30 minutes par la capsaïcine en fin de culture

La figure 2 montre que l'activation de la co-culture pendant 30 minutes par de la capsaïcine à 10µM entraine une augmentation significative de la libération de neuropeptide CGRP (plus 88% ; p<0.0001 par rapport au témoin traité par du milieu contrôle).

Le traitement de la co-culture par la β-endorphine pendant 10 jours avant activation a permis d'inhiber significativement la libération de CGRP (moins 127%; p<0.0001 par rapport au témoin capsaïcine).

Ces résultats valident les conditions de l'étude.

Le traitement de la co-culture pendant 10 jours par le Mix4, aux concentrations C10, C9 ou C8, entraine une inhibition significative de la libération de CGRP (respectivement moins 62% ; p<0.0001, 70% ; p<0.0001 et 75% ; p<0.0001 par rapport au témoin capsaïcine).

Le traitement de la co-culture pendant 10 jours par le Mix5, aux concentrations C10, C9 ou C8, entraine également une inhibition significative de la libération de CGRP (respectivement moins 54% ; p<0.0001 , 68% ; p<0.0001 et 69% ; p<0.0001 par rapport au témoin capsaïcine).

### 1.3. Traitement chronique par le cortisol et les composés

Comme le montre la figure 3, le traitement de la co-culture pendant 10 jours par le cortisol entraine une augmentation non significative de la quantité de neuropeptide CGRP détectée au terme de la culture par rapport au témoin traité par du milieu contrôle (plus 21%).

L'absence de significativité des résultats est probablement due au fait que les surnageants utilisés pour le dosage ont été prélevés après seulement 30 minutes de mise en présence avec les cellules de la co-culture. Le traitement chronique au cortisol a pu entrainer des libérations régulières de neuropeptide pendant les 10 jours de traitement impliquant une diminution de la quantité de neuropeptide stockée dans les vésicules, présentes dans les corps cellulaires et les neurites des neurones sensitifs, et disponible pour la libération. Malgré une synthèse permanente assurée par le corps cellulaire des neurones ce phénomène a probablement entrainé une plus faible libération en fin de culture et la sensibilité du kit ELISA est probablement insuffisante pour détecter de manière robuste ces plus faibles modulations de la libération de neuropeptide.

Le traitement de la co-culture par la β-endorphine pendant les 10 jours de traitement au cortisol a entrainé une diminution significative de la quantité de neuropeptide libérée par rapport au témoin cortisol (moins 55% ; p<0.0001).

Le traitement de la co-culture pendant 10 jours par le Mix4, aux concentrations C10, C9 ou C8, entraine une inhibition significative de la libération de CGRP due au stress cortisol (respectivement moins 24% ; p<0.001, 17% ; p<0.01 et 20% ; p<0.01 par rapport au témoin cortisol).

Le traitement de la co-culture pendant 10 jours par le Mix5, aux concentrations C10, C9 ou C8, entraine une inhibition de la libération de CGRP due au stress cortisol (respectivement moins 14% ; p<0.05, moins 17% ; p<0.01 et 12% par rapport au témoin cortisol). Les résultats sont significatifs pour les concentrations C10 et C9.

### 1.4. Traitement chronique par le cortisol et les composés, puis activées 30 minutes par la capsaïcine en fin de culture

Comme le montre la figure 4, le traitement de la co-culture pendant 10 jours par le cortisol puis l'activation par la capsaïcine en fin de culture n'entraine pas de modulation de la quantité de neuropeptide CGRP.

Ce résultat, contraire à celui attendu, est dû au fait que les surnageants utilisés pour le dosage ont été prélevés après seulement 30 minutes de mise en présence avec les cellules de la co-culture. Le traitement chronique au cortisol a pu entrainer des libérations régulières de neuropeptide pendant les 10 jours de traitement impliquant une diminution de la quantité de neuropeptide stockée dans les vésicules, présentes dans les corps cellulaires et les neurites des neurones sensitifs, et disponible pour la libération. Malgré une synthèse permanente assurée par le corps cellulaire des neurones ce phénomène a probablement entrainé une plus faible libération en fin de culture et la sensibilité du kit ELISA est probablement insuffisante pour détecter ces plus faibles modulations de la libération de neuropeptide.

La présence des mélanges d'actifs ou de la β-endorphine pendant les 10 jours de traitement au cortisol puis pendant l'activation par la capsaïcine a entrainé une diminution de la quantité de neuropeptide libérée par rapport au témoin cortisol.

Cependant le traitement cortisol + capsaïcine n'ayant pas été concluant il est difficile de conclure quant à l'effet des Mix ou de la molécule de référence.

### 2. Immunomarquages

Les sigles # sur les graphiques indiquent la condition qui sert de référentiel pour les tests statistiques ANOVA.

### 2.1. Conditions basales

La figure 5 montre que le traitement de la co-culture par la β-endorphine pendant 10 jours entraine une augmentation non-significative de la survie des neurones sensitifs humains par rapport au témoin traité par du milieu contrôle (plus 10%).

Un traitement de la co-culture par le Mix4 aux concentrations C10, C9 ou C8 pendant 10 jours entraine une augmentation significative de la survie des neurones sensitifs humains (respectivement plus 82% ; p<0.0001, 61% ; p<0.001 et 67% ;p<0.0001 par rapport au témoin traité par du milieu contrôle).

Un traitement de la co-culture par le Mix5 aux concentrations C10, C9 ou C8 pendant 10 jours entraine une augmentation significative mais dose inverse de la survie des neurones sensitifs humains (respectivement plus 92% ; p<0.0001, 78% ; p<0.0001 et 48% ;p<0.01 par rapport au témoin traité par du milieu contrôle).

La figure 6 montre que le traitement de la co-culture par la β-endorphine à 1µM entraine une augmentation significative de la longueur des prolongements des neurones sensitifs humains (plus 28% ; p<0.05 par rapport au témoin traité par du milieu contrôle).

Un traitement de la co-culture par le Mix4 aux concentrations C10, C9 ou C8 pendant 10 jours entraine une augmentation dose dépendante de la longueur des prolongements des neurones sensitifs humains (respectivement plus 24%, 33% ; p<0.01 et 44%; p<0.0001 par rapport au témoin traité par du milieu contrôle). Les résultats sont significatifs pour les concentrations C9 et C8.

Un traitement de la co-culture par le Mix5 aux concentrations C10, C9 ou C8 pendant 10 jours entrainent une augmentation significative et dose dépendante de la longueur des prolongements des neurones sensitifs humains (respectivement plus 83% ; p<0.0001, 121% ; p<0.0001 et 148% ;p<0.0001 par rapport au témoin traité par du milieu contrôle).

La figure 7 montre que le traitement de la co-culture par la β-endorphine à 1µM entraine une augmentation significative de l'expression des récepteurs µ-opioïdes par les neurones sensitifs humains (plus 35% ; p<0.01 par rapport au témoin traité par du milieu contrôle).

Un traitement de la co-culture par le Mix4 aux concentrations C10, C9 ou C8 pendant 10 jours entraine une augmentation significative du taux d'expression des récepteurs µ-opioïdes par les neurones sensitifs humains (respectivement plus 25% ; p<0.05, 25% ; p<0.05 et 28%; p<0.05 par rapport au témoin traité par du milieu contrôle).

Un traitement de la co-culture par le Mix5 aux concentrations C10 ou C9 pendant 10 jours entraine une augmentation dose inverse du taux d'expression des récepteurs µ-opioïdes par les neurones sensitifs humains (respectivement plus 32% ; p<0.01 et 18% par rapport au témoin traité par du milieu contrôle). Le résultat obtenu pour la concentration C10 est significatif. En revanche, à la concentration C8, le mélange d'actif appliqué pendant 10 jours n'entraine pas de modulation du taux d'expression des récepteurs µ- opioïdes.

La figure 8 montre que le traitement de la co-culture par la β-endorphine à 1µM entraine une augmentation significative de l'expression des récepteurs aux glucocorticoïdes par les neurones sensitifs humains (plus 51% ; p<0.01 par rapport au témoin traité par du milieu contrôle).

Un traitement de la co-culture par le Mix4 aux concentrations C10, C9 ou C8 pendant 10 jours entraine une augmentation significative du taux d'expression des récepteurs aux glucocorticoïdes par les neurones sensitifs humains (respectivement plus 77% ; p<0.0001, 63% ; p<0.001 et 75%; p<0.0001 par rapport au témoin traité par du milieu contrôle).

Un traitement de la co-culture par le Mix5 aux concentrations C10, C9 ou C8 pendant 10 jours entraine une augmentation significative et dose inverse du taux d'expression des récepteurs aux glucocorticoïdes par les neurones sensitifs humains (respectivement plus 109% ; p<0.0001, 82% ; p<0.0001 et 67% ; p<0.001 par rapport au témoin traité par du milieu contrôle).

### 2.2. Conditions activées 30 minutes par la capsaïcine en fin de culture

La figure 9 montre que l'activation des cellules par la capsaïcine 30 minutes à la fin de la culture n'entraine pas de modulation de la survie des neurones sensitifs humains par rapport au témoin traité par du milieu contrôle.

Le traitement de la co-culture par la β-endorphine pendant 10 jours puis pendant l'activation par la capsaïcine entraine une augmentation non significative de la survie des neurones sensitifs par rapport aux témoins traités par du milieu contrôle et capsaïcine (plus 22% par rapport au témoin capsaïcine).

Un traitement de la co-culture par le Mix4 aux concentrations C10, C9 ou C8 pendant 10 jours entraine une augmentation dose dépendante de la survie des neurones sensitifs humains (respectivement plus 28%, 56% ; p<0.0001 et 95%; p<0.0001 par rapport au témoin capsaïcine). Les résultats sont significatifs pour les concentrations C9 et C8.

Un traitement de la co-culture par le Mix5 aux concentrations C10, C9 ou C8 pendant 10 jours entraine une augmentation dose inverse de la survie des neurones sensitifs humains (respectivement plus 64% ; p<0.0001, 45% ; p<0.01 et 39% par rapport au témoin capsaïcine). Les résultats sont significatifs pour les concentrations C8, C10 et C9.

La figure 10 montre que le traitement de la co-culture par la capsaïcine 30 minutes avant la fin de la culture n'entraine pas de modulation de la longueur des prolongements des neurones sensitifs humains par rapport au témoin traité par du milieu contrôle.

Le traitement de la co-culture pendant 10 jours puis pendant les 30 minutes de stimulation par la capsaïcine, par la β-endorphine à 1µM entraine une augmentation non significative de la longueur des prolongements des neurones par rapport aux témoins traités par du milieu contrôle ou capsaïcine (plus 22%).

Un traitement de la co-culture par le Mix4 aux concentrations C10, C9 ou C8 pendant 10 jours entraine une augmentation significative et dose dépendante de la longueur des prolongements des neurones sensitifs humains (respectivement plus 37% ; p<0.05, 45% ; p<0.01 et 65% ; p<0.0001 par rapport au témoin capsaïcine).

Un traitement de la co-culture par le Mix5 aux concentrations C10, C9 ou C8 pendant 10 jours entraine une augmentation significative et dose dépendante de la longueur des prolongements des neurones sensitifs humains (respectivement plus 62% ; p<0.0001, 97% ; p<0.0001 et 135% ; p<0.0001 par rapport au témoin capsaïcine).

La figure 11 montre que le traitement de la co-culture par la capsaïcine 30 minutes avant la fin de la culture n'entraine pas de modulation de l'expression des récepteurs µ-opioïdes par les neurones sensitifs humains par rapport au témoin traité par du milieu contrôle.

Le traitement de la co-culture pendant 10 jours, puis pendant les 30 minutes de stimulation par la capsaïcine, par la β-endorphine à 1µM entraine une augmentation significative de l'expression des récepteurs µ- opioïdes par les neurones sensitifs humains (plus 32% ; p<0.01 par rapport au témoin capsaïcine).

Un traitement de la co-culture par le Mix4 aux concentrations C10 ou C9 pendant 10 jours entraine une augmentation non significative du taux d'expression des récepteurs µ-opioïdes par les neurones sensitifs humains (respectivement plus 22% et 23% par rapport au témoin capsaïcine). En revanche, à la concentration C8, le mélange d'actifs ne module pas l'expression des MOR par rapport au témoin capsaïcine.

Un traitement de la co-culture par le Mix5 aux concentrations C10, C9 ou C8 pendant 10 jours entraine une augmentation dose inverse du taux d'expression des récepteurs µ-opioïdes par les neurones sensitifs humains (respectivement plus 43% ; p<0.001, 35% ; p<0.01 et 25% par rapport au témoin capsaïcine). Les résultats sont significatifs pour les concentrations C10 et C9.

La figure 12 montre que le traitement de la co-culture par la capsaïcine 30 minutes avant la fin de la culture entraine une augmentation non significative de l'expression des récepteurs aux glucocorticoïdes par les neurones sensitifs humains (plus 28% par rapport au témoin traité par du milieu contrôle).

Le traitement de la co-culture pendant 10 jours, puis pendant les 30 minutes de stimulation par la capsaïcine, par la β-endorphine à 1µM entraine une augmentation non significative de l'expression des récepteurs aux glucocorticoïdes par les neurones sensitifs humains (plus 24% par rapport au témoin capsaïcine).

Un traitement de la co-culture par le Mix4 aux concentrations C10, C9 ou C8 pendant 10 jours entraine une augmentation significative du taux d'expression des récepteurs aux glucocorticoïdes par les neurones sensitifs humains (respectivement plus 78% ; p<0.0001, 109 %; p<0.0001 et 70% ; p<0.001 par rapport au témoin capsaïcine).

Un traitement de la co-culture par le Mix5 aux concentrations C10, C9 ou C8 pendant 10 jours entraine une augmentation dose inverse du taux d'expression des récepteurs aux glucocorticoïdes par les neurones sensitifs humains (respectivement plus 94% ; p<0.0001, 75% ; p<0.0001 et 37% par rapport au témoin capsaïcine). Les résultats sont significatifs pour les concentrations C10 et C9.

### 2.3. Traitement chronique par le cortisol et les composés

La figure 13 montre que le traitement des cellules par du cortisol à 0.1µM pendant 10 jours entraine une diminution significative de la survie des neurones sensitifs humains par rapport au témoin traité par du milieu contrôle (moins 22% ; p<0.05).

Le traitement de la co-culture par la β-endorphine et du cortisol pendant 10 jours permet de restaurer la survie des neurones sensitifs à un niveau supérieur à celui observé pour la condition témoin traité par du milieu contrôle (plus 41% ; p<0.0001 par rapport au témoin cortisol).

Un traitement de la co-culture par le Mix4 aux concentrations C10, C9 ou C8 pendant 10 jours permet de restaurer significativement la survie des neurones sensitifs humains (respectivement plus 29% ; p<0.01, 45% ; p<0.0001 et 43% ; p<0.0001 par rapport au témoin cortisol). La survie des neurones est supérieure à celle observée dans la condition témoin traité par du milieu contrôle.

Un traitement de la co-culture par le Mix5 aux concentrations C10, C9 ou C8 pendant 10 jours permet de restaurer significativement la survie des neurones sensitifs humains (respectivement plus 43% ; p<0.0001, 67% p<0.0001 et 40% ; p<0.0001 par rapport au témoin cortisol). La survie des neurones est supérieure à celle observée dans la condition témoin traité par du milieu contrôle.

La figure 14 montre que le traitement de la co-culture par du cortisol à 0.1µM pendant 10 jours entraine une diminution significative de la longueur des prolongements des neurones sensitifs humains (moins 42% ; p<0.0001 par rapport au témoin traité par du milieu contrôle).

Le traitement de la co-culture pendant 10 jours par la β-endorphine à 1µM en présence de cortisol permet de restaurer significativement la pousse neuritique des neurones (plus 60% ; p<0.0001 par rapport au témoin cortisol). La longueur des prolongements est supérieure à celle observée dans la condition témoin traité par du milieu contrôle.

Un traitement de la co-culture par le Mix4 aux concentrations C10, C9 ou C8 pendant 10 jours permet de restaurer significativement la pousse neuritique des neurones (respectivement plus 73% ; p<0.0001, 72% ; p<0.0001 et 76% ; p<0.0001 par rapport au témoin cortisol). La longueur des prolongements est supérieure à celle observée dans la condition témoin traité par du milieu contrôle.

Un traitement de la co-culture par le Mix5 aux concentrations C10, C9 ou C8 pendant 10 jours permet de restaurer significativement et de manière dose dépendante la pousse neuritique des neurones (respectivement plus 110% ; p<0.0001, 137% ; p<0.0001 et 167% ; p<0.0001 par rapport au témoin cortisol). La longueur des prolongements est supérieure à celle observée dans la condition témoin traité par du milieu contrôle.

La figure 15 montre que le traitement de la co-culture pendant 10 jours par 0.1µM de cortisol entraine une diminution significative de l'expression des récepteurs µ-opioïdes par les neurones sensitifs humains (moins 27% ; p<0.05 par rapport au témoin traité par du milieu contrôle).

Le traitement de la co-culture pendant 10 jours par la β-endorphine à 1µM en présence de cortisol entraine une augmentation significative de l'expression des récepteurs µ-opioïdes par les neurones sensitifs humains qui atteint un niveau bien supérieur à celui observé pour le témoin traité par du milieu contrôle (plus 92% ; p<0.0001 par rapport au témoin cortisol).

Un traitement de la co-culture par le Mix4 aux concentrations C10, C9 ou C8 pendant 10 jours permet de restaurer significativement le taux d'expression des récepteurs µ-opioïdes par les neurones (respectivement plus 56% ; p<0.0001, 44% ; p<0.001 et 47% ; p<0.0001 par rapport au témoin cortisol). Le taux d'expression des MOR est supérieur à celui observé dans la condition traitée par du milieu contrôle mais inférieur à celui obtenu après traitement par la β-endorphine à 1µM.

Un traitement de la co-culture par le Mix5 aux concentrations C10, C9 ou C8 pendant 10 jours permet de restaurer significativement le taux d'expression des récepteurs µ-opioïdes par les neurones (respectivement plus 66% ; p<0.0001 , 50% ; p<0.0001 et 49% ; p<0.0001 par rapport au témoin cortisol). Le taux d'expression des MOR est supérieur à celui observé dans la condition traitée par du milieu contrôle mais inférieur à celui obtenu après traitement par la β-endorphine à 1µM.

La figure 16 montre que le traitement de la co-culture pendant 10 jours par 0.1µ M de cortisol entraine une augmentation significative de l'expression des récepteurs aux glucocorticoïdes par les neurones sensitifs humains (plus 180% ; p<0.0001 par rapport au témoin traité par du milieu contrôle).

Le traitement de la co-culture pendant 10 jours par la β-endorphine à 1µM en présence de cortisol entraine une diminution significative de l'expression des récepteurs aux glucocorticoïdes par les neurones sensitifs humains (différence de 125% ; p<0.0001 par rapport au témoin cortisol). Cependant le niveau d'expression reste supérieur à celui observé dans la condition traitée par du milieu contrôle.

Un traitement de la co-culture par le Mix4 aux concentrations C10, C9 ou C8 pendant 10 jours permet une diminution significative de l'expression des récepteurs aux glucocorticoïdes par les neurones sensitifs humains rapport au témoin cortisol (différence de 64% ; p<0.0001, 63% ; p<0.0001 et 98% ; p<0.0001 par rapport au témoin cortisol). Cependant le niveau d'expression reste supérieur à celui observé dans la condition traitée par du milieu contrôle.

Un traitement de la co-culture par le Mix5 aux concentrations C10, C9 ou C8 pendant 10 jours permet une diminution de l'expression des récepteurs aux glucocorticoïdes par les neurones sensitifs humains rapport au témoin cortisol (différence de 29%, 37% et 52% ; p<0.05 par rapport au témoin cortisol). Les résultats sont significatifs pour la concentration C8, cependant le niveau d'expression reste supérieur à celui observé dans la condition traitée par du milieu contrôle.

### 2.4. Traitement chronique par le cortisol et les composés et activation 30 minutes par la capsaïcine en fin de culture

La figure 17 montre que le traitement des cellules par du cortisol à 0.1µM pendant 10 jours et pendant l'activation finale par la capsaïcine entraine une diminution significative de la survie des neurones sensitifs humains par rapport au témoin traité par du milieu contrôle (moins 36% ; p<0.05).

Le traitement de la co-culture par la β-endorphine et du cortisol pendant 10 jours et pendant l'activation finale par la capsaïcine permet de restaurer la survie des neurones sensitifs à un niveau comparable au témoin traité par du milieu contrôle (plus 45% ; p<0.001 par rapport au témoin cortisol + capsaïcine).

Un traitement de la co-culture par le Mix4 aux concentrations C10, C9 ou C8 pendant 10 jours permet de restaurer significativement la survie des neurones sensitifs humains rapport au témoin cortisol + capsaïcine (différence de 39% ; p<0.01, 65% ; p<0.0001 et 64% ; p<0.0001). La survie est supérieure au niveau observé dans la condition traitée par du milieu contrôle pour les concentrations C9 et C8.

Un traitement de la co-culture par le Mix5 aux concentrations C10, C9 ou C8 pendant 10 jours permet de restaurer significativement la survie des neurones sensitifs humains rapport au témoin cortisol + capsaïcine (différence de 76% ; p<0.0001, 87% ; p<0.0001 et 57% ; p<0.0001). La survie est supérieure au niveau observé dans la condition traitée par du milieu contrôle.

La figure 18 montre que le traitement de la co-culture par du cortisol à 0.1µ M pendant 10 jours et pendant 30 minutes par la capsaïcine entraine une diminution significative de la longueur des prolongements des neurones sensitifs humains (différence de 32% ; p<0.05 par rapport au témoin traité par du milieu contrôle).

Le traitement de la co-culture pendant 10 jours par la β-endorphine à 1µ M en présence de cortisol et de capsaïcine pendant 30 minutes à la fin de la culture permet de restaurer significativement la pousse neuritique des neurones à un niveau supérieur à celui observé pour le témoin traité par du milieu contrôle (plus 51% ; p<0.0001 par rapport au témoin cortisol + capsaïcine).

Un traitement de la co-culture par le Mix4 aux concentrations C10, C9 ou C8 pendant 10 jours permet de restaurer significativement la pousse neuritique des neurones sensitifs humains rapport au témoin cortisol + capsaïcine (différence de 63% ; p<0.0001, 59% ; p<0.0001 et 67% ; p<0.0001). La longueur des prolongements est supérieure au niveau observé dans la condition traitée par du milieu contrôle.

Un traitement de la co-culture par le Mix5 aux concentrations C10, C9 ou C8 pendant 10 jours permet de restaurer significativement la pousse neuritique des neurones sensitifs humains rapport au témoin cortisol + capsaïcine (différence de 87% ; p<0.0001, 105% ; p<0.0001 et 134% ; p<0.0001). La longueur des prolongements est supérieure au niveau observé dans la condition traitée par du milieu contrôle.

La figure 19 montre que le traitement de la co-culture pendant 10 jours par 0.1µM de cortisol et pendant 30 minutes par la capsaïcine entraine une diminution significative de l'expression des récepteurs µ-opioïdes par les neurones sensitifs humains (différence de 32% par rapport au témoin traité par du milieu contrôle).

Le traitement de la co-culture pendant 10 jours par la β-endorphine à 1µM en présence de cortisol et de capsaïcine pendant les 30 dernières minutes de la culture entraine une restauration significative de l'expression des récepteurs µ-opioïdes par les neurones sensitifs humains (plus 66% ; p<0.0001 par rapport au témoin cortisol + capsaïcine). Le taux d'expression est supérieur à celui observé dans la condition traitée par le milieu contrôle.

Un traitement de la co-culture par le Mix4 aux concentrations C10, C9 ou C8 pendant 10 jours permet une restauration dose inverse de l'expression des récepteurs µ-opioïdes par les neurones sensitifs humains par rapport au témoin cortisol + capsaïcine (différence de 66% ; p<0.0001, 55% ; p<0.0001 et 26%). Le taux d'expression des MOR est significatif et supérieure au niveau observé dans la condition traitée par du milieu contrôle pour les concentrations C9 et C10.

Un traitement de la co-culture par le Mix5 aux concentrations C10, C9 ou C8 pendant 10 jours permet une restauration significative et dose inverse de l'expression des récepteurs µ-opioïdes par les neurones sensitifs humains par rapport au témoin cortisol + capsaïcine (différence de 80% ; p<0.0001 , 52% ; p<0.0001 et 33% ; p<0.05). Le taux d'expression des MOR est supérieure au niveau observé dans la condition traitée par du milieu contrôle pour les concentrations C10 et C9.

La figure 20 montre que le traitement de la co-culture pendant 10 jours par 0.1µM de cortisol et pendant 30 minutes en fin de culture par de la capsaïcine entraine une augmentation significative de l'expression des récepteurs aux glucocorticoïdes par les neurones sensitifs humains (différence de 154% ; p<0.001 par rapport au témoin traité par du milieu contrôle).

Le traitement de la co-culture pendant 10 jours par la β-endorphine à 1µM en présence de cortisol et de capsaïcine pendant 30 minutes à la fin de la culture entraine une diminution significative de l'expression des récepteurs aux glucocorticoïdes par les neurones sensitifs humains (moins 93% ; p<0.0001 par rapport au témoin cortisol + capsaïcine). Cependant le niveau d'expression reste supérieur à celui observé dans la condition traitée par du milieu contrôle.

Un traitement de la co-culture par le Mix4 aux concentrations C10, C9 ou C8 pendant 10 jours permet une restauration dose dépendant de l'expression des récepteurs aux glucocorticoïdes par les neurones sensitifs humains par rapport au témoin cortisol + capsaïcine (différence de 35% ; p<0.01 , 60% ; p<0.01 et 99% ; p<0.0001). Cependant le niveau d'expression reste supérieur à celui observé dans la condition non traitée. Les résultats sont significatifs pour les concentrations C9 et C8.

Un traitement de la co-culture par le Mix5 aux concentrations C10, C9 ou C8 pendant 10 jours permet une restauration dose dépendante de l'expression des récepteurs aux glucocorticoïdes par les neurones sensitifs humains par rapport au témoin cortisol + capsaïcine (respectivement différence de 26%, 61% ; p<0.001 et 80% ; p<0.0001). Cependant le niveau d'expression reste supérieur à celui observé dans la condition non traitée. Les résultats sont significatifs pour les concentrations C9 et C8.

### Conclusions

### • Libération de CGRP :

Le Mix 4, aux trois concentrations évaluées, appliqué pendant 10 jours induit une augmentation non significative de la libération de neuropeptide CGRP en absence d'activation des cellules. Cette augmentation est due probablement à l'augmentation de la survie des neurones au contact de ce mix, en conditions basales. En présence d'un stress capsaïcine ou cortisol, le mélange d'actifs, aux trois concentrations évaluées permet d'inhiber significativement la libération de neuropeptides CGRP. Le Mix 4 aux trois concentrations évaluées ne permet cependant pas d'inhiber totalement la libération de CGRP induite par la capsaïcine.

Le Mix 5, aux trois combinaisons de concentrations testées, et appliqué pendant 10 jours, induit une augmentation significative de la libération de neuropeptide CGRP en absence d'autres stimulations des neurones. Cette augmentation est due probablement à l'augmentation de la survie des neurones au contact de ce mix, en conditions basales. En présence d'un stress capsaïcine ou cortisol, le mélange d'actifs permet d'inhiber significativement la libération de neuropeptides CGRP aux trois concentrations évaluées. Il ne permet cependant pas d'inhiber totalement la libération de CGRP induite par la capsaïcine. Cet effet est également observé après activation finale par la capsaïcine.

### • Viabilité des neurones sensitifs :

Le Mix 4, aux trois concentrations évaluées, appliqué pendant 10 jours permet d'améliorer la survie des neurones sensitifs. Cet effet n'est pas atténué par l'activation finale par la capsaïcine.

Le Mix 4, aux trois concentrations évaluées, appliqué pendant 10 jours permet d'inhiber la toxicité due au traitement des neurones par le cortisol. Cet effet est également observé après activation finale par la capsaïcine.

Le Mix 5, aux trois concentrations évaluées, appliqué pendant 10 jours permet d'améliorer la survie des neurones sensitifs. Cependant l'effet est moins important pour les concentrations C9 ou C8. Cet effet n'est pas atténué par l'activation finale par la capsaïcine.

Le Mix 5 appliqué pendant 10 jours permet d'inhiber la toxicité due au traitement des neurones par le cortisol. Cet effet est également observé après activation finale par la capsaïcine.

### • Pousse neuritique :

Le Mix 4 et le Mix 5, aux trois concentrations évaluées, appliqués pendant 10 jours permettent d'activer la pousse neuritique des neurones sensitifs. Cet effet n'est pas atténué par l'activation finale par la capsaïcine.

Le Mix 4 et le Mix 5, aux trois concentrations évaluées, appliqués pendant 10 jours, permettent d'inhiber la toxicité due au traitement des neurones par le cortisol (restauration de la pousse neuritique). Cet effet est également observé après activation finale par la capsaïcine.

### • Expression des récepteurs µ-opioïdes

Le Mix 4 et le Mix 5, aux trois concentrations évaluées, appliqués pendant 10 jours, permettent d'augmenter le taux d'expression des récepteurs µ-opioïdes. Cet effet n'est pas atténué par l'activation finale par la capsaïcine. Le Mix 4 et le Mix 5, aux trois concentrations évaluées, appliqués pendant 10 jours, permettent d'inhiber l'effet délétère du cortisol sur le taux d'expression des MOR. Cet effet est également observé après activation finale par la capsaïcine.

### • Expression des récepteurs aux glucocorticoïdes

Le Mix 4 et le Mix 5, aux trois concentrations évaluées, appliqués pendant 10 jours, entrainent une augmentation du taux d'expression des récepteurs aux glucocorticoïdes par les neurones sensitifs. Cet effet n'est pas impacté par l'activation finale par la capsaïcine. Le Mix 4 et le Mix 5, aux trois concentrations évaluées, appliqués pendant 10 jours, permettent d'inhiber l'effet délétère du cortisol sur le taux d'expression des GR. Cet effet est également observé après activation finale par la capsaïcine.

Le Mix 4 possède donc des propriétés apaisantes permettant de contrer l'effet irritant de la capsaïcine. De plus il permet d'améliorer la physiologie des neurones en augmentant leur survie, en activant la pousse neuritique et en augmentant l'expression des récepteurs µ-opioïdes. Ce Mix permet de protéger les neurones de l'effet délétère d'un traitement chronique par le cortisol.

Tout comme le Mix4, le Mix 5 possède des propriétés apaisantes permettant de contrer l'effet irritant de la capsaïcine. De plus il permet d'améliorer la physiologie des neurones en augmentant leur survie, en activant la pousse neuritique et en augmentant l'expression des récepteurs µ-opioïdes. Ce Mix permet de protéger les neurones de l'effet délétère d'un traitement chronique par le cortisol.

Exemple d'une composition selon l'invention

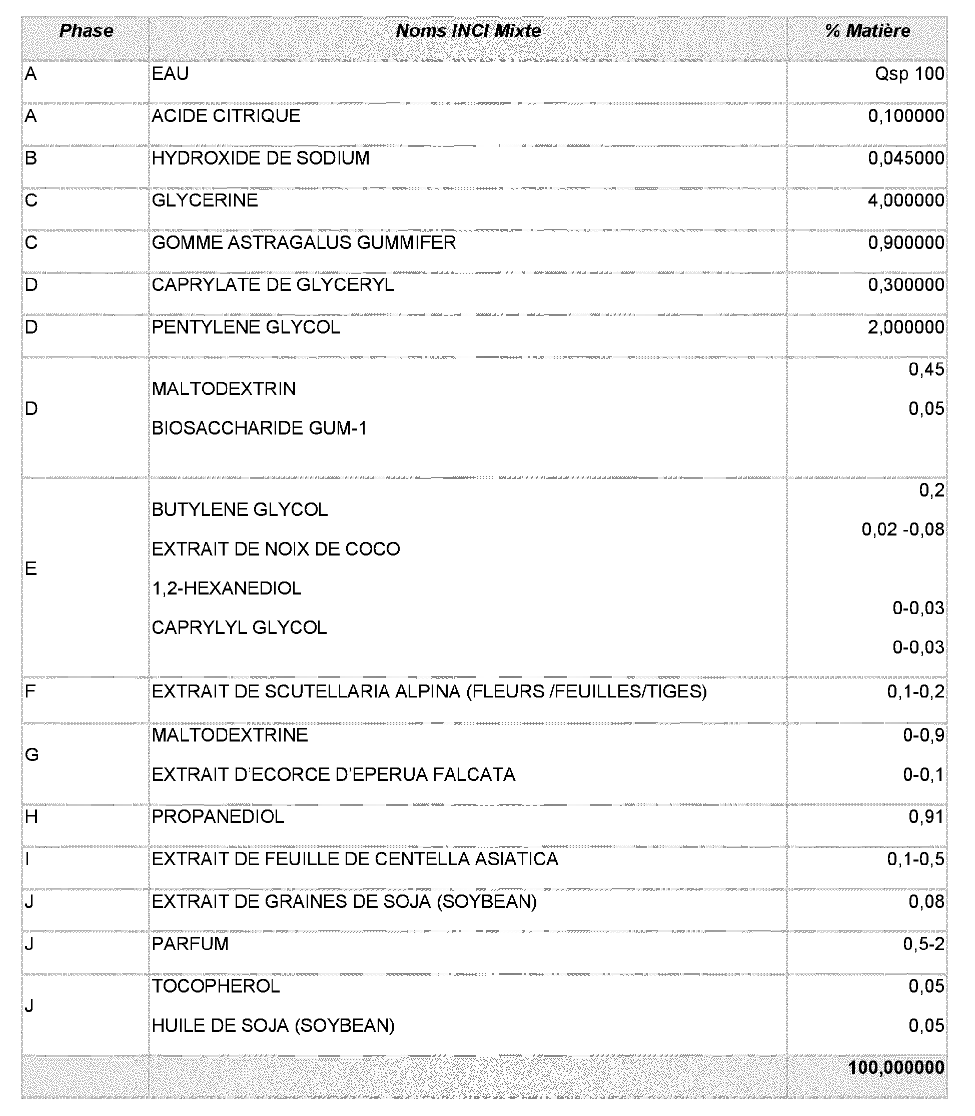

## Revendications

1. Composition neurocosmétique à usage topique, comprenant :
a) Une quantité efficace d'un extrait de fruit de *Cocos nucifera, et*
b) Une quantité efficace d'un extrait de *Scutellaria alpina.*

2. Composition neurocosmétique selon la revendication 1, **caractérisée en ce qu'**elle contient en outre une quantité efficace d'un extrait *d'Eperua falcata.*

3. Composition neurocosmétique selon l'une quelconque des revendications 1 ou 2, **caractérisée en ce qu'**elle contient :
a) entre 0.01 et 1% en poids de farine de fruit de *Cocos nucifera,*
b) entre 0.1 et 0.5% en poids d'un extrait de fleurs, de tiges et/ou de feuilles de *Scutellaria alpina,*
et optionnellement :
c) entre 0 et 0.5% en poids d'un extrait d'écorce *d'Eperua falcata.*

4. Utilisation cosmétique non thérapeutique de la composition neurocosmétique selon l'une quelconque des revendications 1 à 3, pour augmenter la survie des neurones sensoriels cutanés, pour augmenter la pousse neuritique des neurones cutanés, ou pour augmenter l'expression des récepteurs µ-opioïdes tels que les récepteurs de la beta-endorphine.

5. Utilisation cosmétique non thérapeutique de la composition neurocosmétique selon la revendication 4, sur des peaux soumises à un stress chronique ou en l'absence de stress.

6. Utilisation cosmétique non thérapeutique de la composition neurocosmétique selon la revendication 4, pour prévenir les effets délétères d'un stress aigu ou chronique sur la peau et/ou pour renforcer la résistance de la peau aux stress chroniques ou aigus.

7. Utilisation cosmétique non thérapeutique de la composition neurocosmétique selon la revendication 4, pour améliorer l'homéostasie et/ou pour apporter une sensation de bien-être.

8. Utilisation cosmétique non thérapeutique de la composition neurocosmétique selon la revendication 4, pour diminuer dans les neurones de la peau l'expression des récepteurs aux glucocorticoïdes, tels que les récepteurs du cortisol, notamment en présence d'un stress chronique.

9. Utilisation cosmétique non thérapeutique de la composition neurocosmétique selon la revendication 4, pour améliorer l'hydratation de la peau et/ou améliorer la fonction barrière de la peau et/ou améliorer l'éclat de la peau.

10. Utilisation cosmétique non thérapeutique de la composition neurocosmétique selon la revendication 4, pour son effet anti-âge, antiride, ou pour renforcer la fermeté de la peau.
